# FASCICULE DE BREVET EUROPEEN

(11) **EP 1 991 557 B1**
(45) Date de publication et mention de la délivrance du brevet: **20.01.2010**
(21) Numéro de dépôt: 07731037.3
(22) Date de dépôt: 23.02.2007
(51) Int. Cl.: C07H 11/04, A61K 31/7024

(54) **MANNOSYL-1 PHOSPHATES, PROCEDE DE PREPARATION ET UTILISATION EN THERAPEUTIQUE, NOTAMMENT VIS-A-VIS DU SYNDROME CDG-IA**
MANNOSYL-1 PHOSPHATE, ZUBEREITUNGSVERFAHREN UND THERAPEUTISCHE VERWENDUNG, IM BESONDEREN GEGEN DAS CDG-IA-SYNDROM
MANNOSYL-1 PHOSPHATES, PREPARATION METHOD AND THERAPEUTIC USE, IN PARTICULAR AGAINST THE CDG-IA SYNDROME

(30) Priorité: 24.02.2006 FR 0601646
(43) Date de publication de la demande: 19.11.2008
(73) Titulaire: OE Operations, 92800 Puteaux (FR); Université René Descartes (Paris V), 75006 Paris (FR); Centre National de la Recherche Scientifique - CNRS, 75016 Paris Cedex 16 (FR)
(72) Inventeur: GRAVIER-PELLETIER, Christine, F-91540 Mennecy (FR); HARDRE, Renaud, F-75270 Paris Cedex 06 (FR); KHALED, Amira, F-75270 Paris Cedex 06 (FR); LE MERRER, Yves, F-94380 Bonneuil-sur-Marne (FR)
(74) Mandataire: Bertrand, Didier
(86) Numéro de dépôt international: PCT/FR2007/000332
(87) Numéro de publication internationale: WO 2007/096532

(56) Documents cités:
- WO-A-03/104247
- HWANG, YOUSANG ET AL: "Efficient Synthesis of Phosphorylated Prodrugs with Bis(POM)-phosphoryl Chloride" ORGANIC LETTERS , 6(10), 1555-1556 CODEN: ORLEF7; ISSN: 1523-7060, 2004, XP002400126
- EKLUND, ERIK A. ET AL: "Hydrophobic Man-1-P derivatives correct abnormal glycosylation in Type I congenital disorder of glycosylation fibroblasts" GLYCOBIOLOGY , 15(11), 1084-1093 CODEN: GLYCE3; ISSN: 0959-6658, 2005, XP002400127 cité dans la demande
- RUTSCHOW S ET AL: "Membrane-Permeant derivatives of mannose-1-phosphate" BIOORGANIC & MEDICINAL CHEMISTRY, ELSEVIER SCIENCE LTD, GB, vol. 10, 2002, pages 4043-4049, XP002259386 ISSN: 0968-0896 cité dans la demande
- P.J. GAREGG, J. HANSSON,A-C HELLAND, S. OSCARSON: "Formation of anomeric phosphodiester linkages using H-phosphonate acceptors" TETRAHEDRON LETTERS, vol. 40, 1999, pages 3049-3052, XP002400475
- R, ROY, F.D. TROPPER, C.GRAND-MAITRE: "Syntheses of glycosyl phosphate by transfer catalysis" CANADIAN JOURNAL OF CHEMISTRY, vol. 69, 1991, pages 1462-1467, XP009072961
- PLANTE, OBADIAH J. ET AL: "Formation of .beta.-Glucosamine and .beta.-Mannose Linkages Using Glycosyl Phosphates" ORGANIC LETTERS , 2(24), 3841-3843 CODEN: ORLEF7; ISSN: 1523-7060, 2000, XP002400128
- GARCIA, BRIAN A. ET AL: "Synthesis of Glycosyl-1-phosphates via Dehydrative Glycosylation" ORGANIC LETTERS , 2(14), 2135-2138 CODEN: ORLEF7; ISSN: 1523-7060, 2000, XP002400129
- PLANTE, OBADIAH J. ET AL: "Oligosaccharide Synthesis with Glycosyl Phosphate and Dithiophosphate Triesters as Glycosylating Agents" JOURNAL OF THE AMERICAN CHEMICAL SOCIETY , 123(39), 9545-9554 CODEN: JACSAT; ISSN: 0002-7863, 2001, XP002400130
- WATANABE Y ET AL: "Synthesis of 2,6-di-o-alpha-d-mannopyranosylphosphatidy l-d-myo-inosi tol. Utilization of glycosylation and phosphorylation based on phosphite chemistry" TETRAHEDRON, ELSEVIER SCIENCE PUBLISHERS, AMSTERDAM, NL, vol. 53, no. 3, 20 janvier 1997 (1997-01-20), pages 903-918, XP004105190 ISSN: 0040-4020

## Description

### Domaine de l'invention

La présente invention a trait à une nouvelle solution technique pour le traitement du syndrome CDG (désordres congénitaux de la glycosylation) de type I et plus particulièrement de type la Selon l'invention, cette nouvelle solution fait appel à des dérivés de mannosyl-1 phosphate, à savoir les mono(mannopyranosyl-1), di(mannopyranosyl-1) et tri(mannopyranosyl-1) phosphates dont les formules sont données ci-après. En bref l'invention concerne plus précisément :
- les mono(mannopyranosyl-1) phosphates de formule I, les di(mannopyranosyl-1) phosphates de formule II et les tri(mannopyranosyl-1) phosphates de formule III, en tant que médicaments utilisables vis-à-vis du syndrome CDG-I et plus particulièrement vis-à-vis du syndrome CDG-la,
- l'utilisation en thérapeutique desdits mono(mannopyranosyl-1) phosphates de formule I, di(mannopyranosyl-1) phosphates de formule II et tri(mannopyranosyl-1) phosphates de formule III, dans le traitement du syndrome CDG-I et plus particulièrement celui du syndrome CDG-Ia,
- les mono(mannopyranosyl-1 ) phosphates de formule I, les di(mannopyranosyl-1) phosphates de formule II et les tri(mannopyranosyl-1) phosphates de formule III', en tant que produits industriels nouveaux, et
- un procédé de préparation desdits produits nouveaux.

### Art antérieur

Le syndrome CDG est un groupe de maladies autosomiques récessives touchant la synthèse des glycoprotéines. Ces maladies, qui sont liées à différents déficits enzymatiques, se traduisent par des atteintes neurologiques auxquelles peuvent être associées des atteintes multiviscérales. Leur classification est fondée sur le niveau de l'étape qui limite la glycosylation. Pour le syndrome CDG-I, qui est statistiquement celui que l'on rencontre le plus souvent, l'atteinte, qui se traduit par une N-glycosylation intracellulaire insuffisante, se situe en amont du transfert de l'oligosaccharide sur la chaîne peptidique, en revanche pour le syndrome CDG-II elle se situe en aval dudit transfert. Parmi les cas de syndrome CDG-I, le plus fréquent (70 % desdits cas) est le syndrome CDG-Ia ; il s'agit d'une maladie rare touchant environ 500 personnes dans le monde et qui est caractérisée par un déficit en activité phosphomannomutase (PMM) et des mutations sur le gène de PMM2 (i. e. le gène exprimant la phosphomannomutase 2) situé en 16p13, les autres CDG-I et CDG-II ne concernant qu'un nombre relativement restreint de cas.

Le métabolisme intracellulaire est schématisé par Muus U. et al., Eur. J. Org. Chem., 2004; 1228-1235 comme suit :

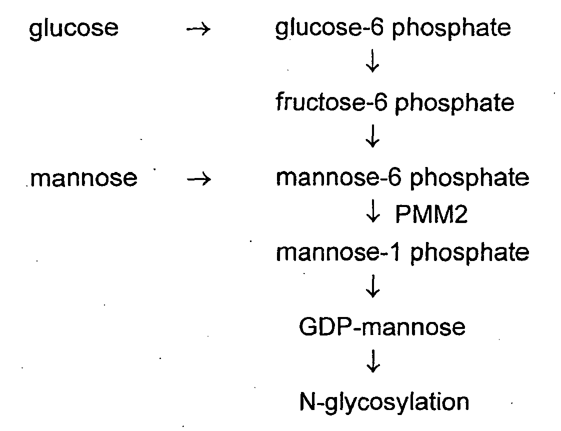

Dans le cas du syndrome CDG-I, et notamment celui du syndrome CDG-Ia, un défaut au niveau de l'activité de PMM2 entraîne un défaut ou une insuffisance de la N-glycosylation intracellulaire.

Pour remédier à un tel défaut de métabolisme, il est pertinent de fournir à la cellule du mannose-1 phosphate (en abrégé : Man-1 P). Or le Man-1 P administré par voie orale ou injectable est dégradé par les enzymes des liquides corporels extracellulaires, et le Man-1 P non décomposé, qui peut atteindre le niveau cellulaire où il est nécessaire, ne peut pas pénétrer la paroi cellulaire en raison de sa forte polarité due à la présence de deux groupes OH acides présents sur l'atome de phosphore, comme cela est rappelé par Rutschow S. et al., Bioorg. Med. Chem., 2002; 10: 4043-4049.

Parmi les solutions envisagées pour diminuer la polarité de Man-1 P, on connaît celles décrites dans :
- l'article de Rutschow S. *et al.* précité, qui correspond à et est développé dans WO 2003/104247 A (Marquardt T. et al.),
- l'article de Muus U. et al. précité, et
- l'article de Eklund E. A. et al., Glycobiology, 2005; 15 (No. 11): 1084-1093,
qui ont trait à des dérivés de mono(mannopyranosyl-1) phosphate, dans lesquels (i) les deux groupes OH acides du reste phosphate sont chacun avantageusement protégés par un groupe protecteur de la fonction acide PO-OH qui est éliminable, en général le même groupe R = R' est utilisé pour chaque OH acide (PO-OH), et (ii) au moins un groupe OH du reste mannopyranosyle est également protégé par un groupe OH-protecteur éliminable, en général les 4 groupes OH dudit reste mannopyranosyle sont protégés.

L'ensemble Rutschow S. et al./WO 2003/104247 A décrit, en tant qu'agents thérapeutiques vis-à-vis du syndrome CDG-Ia, des mono(α-D-mannopyranosyl-1) phosphates extracellulairement stables, capables de traverser la paroi cellulaire pour fournir une source au niveau intracellulaire de Man-1 P et ayant une structure correspondant à la formule I ci-après, dans laquelle R₁₁, R₁₂, R₁₃ et R₁₄, identiques ou différents, représentent chacun un groupe alkylcarbonyle, arylcarbonyle, alkyloxycarbonyle ou aryloxycarbonyle, R₁₁, R₁₂ et R₁₃ pouvant représenter en outre H, d'une part, et R et R', identiques ou différents, représentent chacun un groupe OH ou un groupe oxyméthylèneoxycarbonylalkyle [i. e. O-CH₂-O-CO-Alk], d'autre part, les groupes alkyle étant à chaîne hydrocarbonée linéaire ou ramifiée en C₁-C₂₀ et les groupes aryle étant des restes hydrocarbonés aromatiques éventuellement substitués.

Il se trouve que les composés dudit ensemble Rutschow S. et al./WO 2003/104247 A, (i) sont relativement stables dans les liquides corporels extracellulaires, (ii) sont susceptibles de traverser au moins partiellement la paroi cellulaire, à l'exception des composés R = R' = OH trop polaires, mais (iii) sont cytotoxiques en ce sens que sous l'action d'estérases intracellulaires le groupe R = oxyméthylèneoxycarbonylalkyle précité fournit du formaldéhyde (voir à cet effet le schéma 1 de la page 4045 de l'article de Rutschow S. *et al.*). Ainsi, plus la capacité de ces produits à traverser la paroi cellulaire est grande, plus leur toxicité intracellulaire est élevée. Tel est le cas des produits suivants dudit ensemble, à savoir les :
- CP1 :: di(pivaloyloxyméthyle) (2,3,4,6-tétra-O-acétyl)-α-D-mannopyranosyl-1] phosphate,
- CP2 :: di(pivaloyloxyméthyle) (2,3,4,6-tétra-O-butyryl-α-D-mannopyranosyl-1) phosphate (composé 11 dudit ensemble),
- CP3 :: di(pivaloyloxyméthyle) (2,3,4,6-tétra-O-pivaloyl-α-D-mannopyranosyl-1) phosphate (composé 13 dudit ensemble), et
- CP4 :: di(pivaloyloxyméthyle) [2,3,4,6-tétra-O-(isopropylcarbonyl)α-D-mannopyranosyl-1] phosphate (composé 15 dudit ensemble).

L'article de Eklund E. A. *et al.* décrit des composés similaires utiles vis-à-vis du syndrome CDG-Ia, à savoir les :
- CP5 :: di(acétyloxyméthyle) (2,3,4,6-tétra-O-acétyl)-α-D-mannopyranosyl-1) phosphate [composé 5 (référencé C-I) dudit article], et
- CP6 :: di(acétyloxyméthyle) (2,3,4,6-tétra-O-éthyloxycarbonyl)-α-Dmannopyranosyl-1) phosphate [composé 10 (référencé C-II) dudit article].

L'article de Muus U. *et al.* propose une autre solution technique mettant en oeuvre un ester cyclique de l'acide phosphorique de structure cyclosaligényl-mannopyranosyl-1 phosphate : 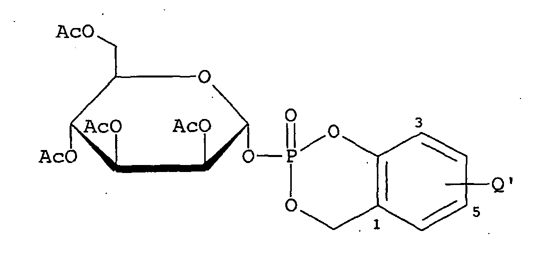 dans laquelle Q' est H, 5-CI, 3-Me ou 3,5-diMe.

Enfin sur le plan de la synthèse, on connaît
- de l'article de Colowick S. P., J. Biol. Chem., 1938: 124; 557-558, la préparation de tri[(2,3,4,6-tétra-O-acétyl)-α-D-mannopyranosyl-1] phosphate par réaction de 1-bromo-(2,3,4,6-tétra-O-acétyl)-α-D-mannopyranose avec Ag₃PO₄, cet article ne décrivant pas l'utilisation de ce produit en tant que médicament; et
- de l'article de Eklund E. A. *et al*. précité, l'obtention d'un dérivé de mono(mannopyranosyl-1) phosphate par réaction de (2,3,4,6-tétra-O-acétyl)-α-D-mannopyranose, en présence de Ag₂CO₃, avec le dibenzyle phosphate [HOP(=O)(OCH₂C₆H₅)₂].

### But de l'invention

Selon l'invention, on se propose de fournir des dérivés de Man-1 P qui soient (i) essentiellement stables dans les liquides corporels extracellulaires, (ii) capables de traverser substantiellement la paroi cellulaire, et (iii) intracellulairement moins toxiques ou moins cytotoxiques que les meilleurs produits de l'art antérieur qui sont représentés par les composés CP1 à CP6 précités.

Le cas échéant, la diminution, que l'on recherche, de la toxicité intracellulaire due en particulier aux produits de dégradation peut résulter d'un compromis entre la capacité de pénétration desdits dérivés à travers la paroi cellulaire et la toxicité intracellulaire de leurs produits de dégradation enzymatique.

On se propose également d'utiliser, comme nouveaux médicaments, de tels dérivés de Man-1 P qui, en tant que prodrogues de Man-1 P, vont intervenir chacun comme une source intracellulaire de Man-1 P, en vue de produire par dégradation enzymatique intracellulaire le Man-1 P nécessaire dans le syndrome CDG-I, et plus particulièrement dans le syndrome CDG-Ia, afin de restaurer la N-glycosylation intracellulaire requise.

On se propose enfin de fournir un procédé de préparation desdits dérivés de Man-1 P, qu'ils soient de structure mono(mannopyranosyl-1) phosphate, di(mannopyranosyl-1) phosphate ou tri(mannopyranosyl-1) phosphate.

### Objet de l'invention

Selon un aspect de l'invention, on préconise une composition pour une utilisation comme médicament, ladite composition renfermant en association avec un excipient physiologiquement acceptable, une substance active choisie parmi l'ensemble constitué par :
(α) les mono(α-D-mannopyranosyl-1) phosphates de formule I: 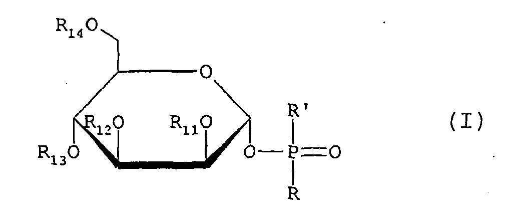 dans laquelle
   R₁₁, R₁₂, R₁₃ et R₁₄, identiques ou différents, représentent chacun l'atome d'hydrogène ou un groupe OH-protecteur,
   R et R', identiques ou différents, représentent chacun
   - un groupe aryloxy en C₆-C₁₀ (notamment phénoxy, 1-naphtyloxy ou 2-naphtyloxy) susceptible d'être substitué par un ou plusieurs groupes alkyle en C₁-C₅, alkoxy en C₁-C₅, halogéno, CF₃ et/ou nitro,
   - un groupe arylalkylèneoxy (tel que notamment OCH₂CH₂C₆H₅, OCH₂C₆H₅, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyl en C₁-C₅, alkoxy en C₁-C₅, halogéno, CF₃ et/ou nitro,
   - un groupe de structure :
      -O-CH(CH₃)-O-CO-alkyle, ou
      -O-CH(CH₃)-O-CO-O-alkyle
      où le reste alkyle est en C₁-C₅,
   - un groupe -O-CH₂-CH(OH)-CH₂OH, où les groupes OH peuvent être protégés,
   - un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
   - un groupe aminoacide de structure VIIa : 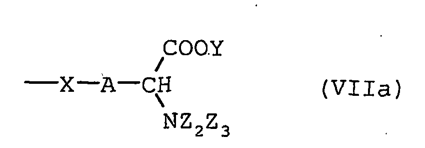 où
      X est -O-, -S- ou -NZ₁-,
      Y représente H ou un groupe alkyle en C₂-C₅,
      A est un groupe alkylène, phénylène ou phénylalkylène, (où chaque groupe alkylène est en C₁-C₅),
      Z₁ est H, un groupe alkyle en C₁-C₅ ou un groupe N-protecteur,
      Z₂ et Z₃, identiques ou différents, représentent chacun H, un groupe alkyle en C₁-C₅, ou un groupe N-protecteur, ou
   - un groupe aminoacide de structure VIIb : 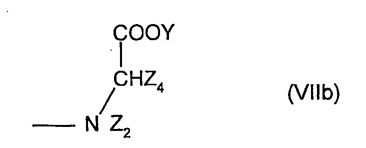 où
      Y représente H ou un groupe alkyle en C₂-C₅,
      Z₄ est un groupe alkylène, phénylène ou phénylalkylène, (où chaque groupe alkylène est en C₁-C₅), ou une chaine latérale des aminoacides naturels, protégée ou non par un groupement protecteur,
      Z₂ représente H, un groupe alkyle en C₁-C₅, ou un groupe N-protecteur ;
(β) les di(α-D-mannopyranosyl-1) phosphates de formule II : 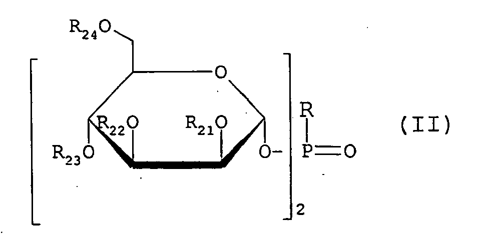 dans laquelle
   R₂₁ , R₂₂, R₂₃ et R₂₄, identiques ou différents, représentent chacun l'atome d'hydrogène ou un groupe OH-protecteur, et
   R représente
   - un groupe OH,
   - un groupe alkoxy en C₁-C₂₀ (de préférence en C₁-C₅),
   - un groupe aryloxy en C₆-C₁₀ susceptible d'être substitué par un ou plusieurs groupes alkyle en C₁-C₅, alkoxy en C₁-C₅, halogéno, CF₃ et/ou nitro,
   - un groupe arylalkylèneoxy (notamment benzyloxy, phényléthyloxy, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
   - un groupe de structure:
      -O-CH(Q)-O-CO-alkyle, ou
      -O-CH(Q)-O-CO-O-alkyle
      où Q est H ou CH₃, et le reste alkyle est en C₁-C₅,
   - un groupe O-CH₂-CH(OH)-CH₂OH, où les groupes OH peuvent être protégés,
   - un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
   - un groupe aminoacide de structure VIIa : 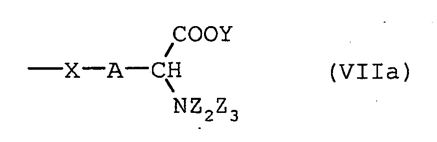 où
      X est -O-, -S- ou -NZ₁-,
      Y représente H ou un groupe alkyle en C₂-C₅,
      A est un groupe alkylène, phénylène ou phénylalkylène, (où chaque groupe alkylène est en C₁-C₅),
      Z₁ est H, un groupe alkyle en C₁-C₅ ou un groupe N-protecteur, et
      Z₂ et Z₃, identiques ou différents, représentent chacun H, un groupe alkyle en C₁-C₅, ou un groupe N-protecteur ;
   - un groupe aminoacide de structure VIIb : 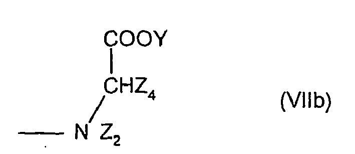 où
      Y représente H ou un groupe alkyle en C₂-C₅,
      Z₄ est un groupe alkylène, phénylène ou phénylalkylène, (où chaque groupe alkylène est en C₁-C₅), ou une chaine latérale des aminoacides naturels, protégée ou non par un groupement protecteur,
      Z₂ représente H, un groupe alkyle en C₁-C₅, ou un groupe N-protecteur ;
(γ) les tri(α-D-mannopyranosyl-1) phosphates de formule III : 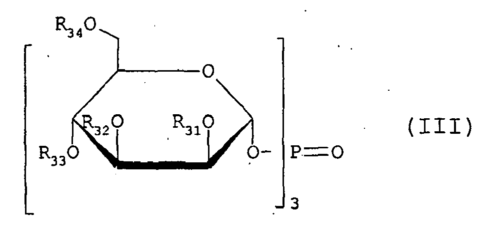 dans laquelle
   R₃₁, R₃₂, R₃₃ et R₃₄, identiques ou différents, représentent chacun un atome d'hydrogène ou un groupe OH-protecteur ; et
(δ) leurs mélanges.

Dans cette composition, ledit ingrédient actif est présent selon une quantité thérapeutiquement efficace et intervient en tant que source intracellulaire de Man-1 P. Grâce aux groupes R prévus et aux structures moléculaires envisagées, qu'elles soient mono, di ou a fortiori tri(mannopyranosyl-1) phosphate, elles sont intracellulairement très peu toxiques ou cytotoxiques, car sous l'action des estérases intracellulaires, elles ne conduisent pas à la formation de formaldéhyde qui est très toxique, mais à la formation d'autres molécules, relativement moins toxiques. Globalement, la composition selon l'invention permet d'obtenir des dérivés de Man-1 P qui sont stables dans les liquides corporels extracellulaires et qui peuvent traverser la paroi cellulaire, mais surtout qui sont intracellulairement moins toxiques ou cytotoxiques que les meilleurs produits de l'art antérieur qui sont représentés par les composés CP1 à CP6 précités.

Selon un autre aspect de l'invention, on préconise l'utilisation d'un dérivé de (mannosyl-1) phosphate, ladite utilisation étant caractérisée en ce que l'on fait appel à une substance intervenant en tant que source intracellulaire de Man-1 P, qui est choisie parmi l'ensemble constitué par les composés
(α) mono(α-D-mannopyranosyl-1) phosphates de formule I,
(β) di(α-D-mannopyranosyl-1) phosphates de formule II,
(γ) tri(α-D-mannopyranosyl-1) phosphates de formule III, et
(δ) leurs mélanges,
pour la préparation d'un médicament destiné à un usage en thérapeutique vis-à-vis du syndrome CDG-I, et en particulier vis-à-vis du syndrome CDG-la.

Selon encore un autre aspect de l'invention, on fournit en tant que produit industriel nouveau un dérivé de (mannosyl-1) phosphate, utilisable vis-à-vis du syndrome CDG-I et en particulier vis-à-vis du syndrome CDG-Ia, caractérisé en ce qu'il est choisi parmi l'ensemble constitué par :
(α) les mono(α-D-mannopyranosyl-1) phosphates de formule I : 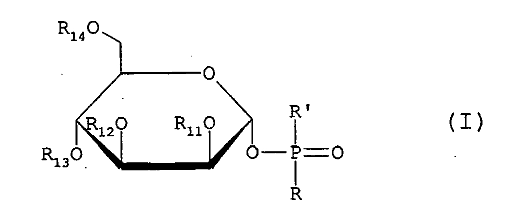 dans laquelle
   R₁₁, R₁₂, R₁₃ et R₁₄, identiques ou différents, représentent chacun l'atome d'hydrogène ou un groupe OH-protecteur,
   R et R', identiques ou différents, représentent chacun
   - un groupe aryloxy en C₆-C₁₀ (notamment phénoxy, 1-naphtyloxy ou 2-naphtyloxy) susceptible d'être substitué par un ou plusieurs groupes alkyle en C₁-C₅, alkoxy en C₁-C₅, halogéno, CF₃ et/ou nitro,
   - un groupe arylalkylèneoxy (notamment OCH₂CH₂C₆H₅, OCH₂C₆H₅, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
   - un groupe de structure :
      -O-CH(CH₃)-O-CO-alkyle, ou
      -O-CH(CH₃)-O-CO-O-alkyle
      où le reste alkyle est en C₁-C₅,
   - un groupe -O-CH₂-CH(OH)-CH₂OH, où les groupes OH peuvent être protégés,
   - un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
   - un groupe aminoacide de structure VIIa : 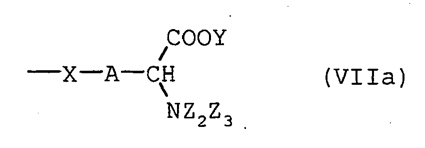 où
      X est -O-, -S- ou -NZ₁-,
      Y représente H ou un groupe alkyle en C₂-C₅,
      A est un groupe alkylène, phénylène ou phénylalkylène, (où chaque groupe alkylène est en C₁-C₅),
      Z₁ est H, un groupe alkyle en C₁-C₅ ou un groupe N-protecteur, et
      Z₂ et Z₃, identiques ou différents, représentent chacun H, un groupe alkyle en C₁-C₅, ou un groupe N-protecteur ;
   - un groupe aminoacide de structure VIIb : 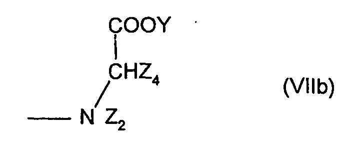 où
      Y représente H ou un groupe alkyle en C₂-C₅,
      Z₄ est un groupe alkylène, phénylène ou phénylalkylène, (où chaque groupe alkylène est en C₁-C₅), ou une chaine latérale des aminoacides naturels, protégée ou non par un groupement protecteur,
      Z₂ représente H, un groupe alkyle en C₁-C₅, ou un groupe N-protecteur ;
(β) les di(α-D-mannopyranosyl-1) phosphates de formule II: 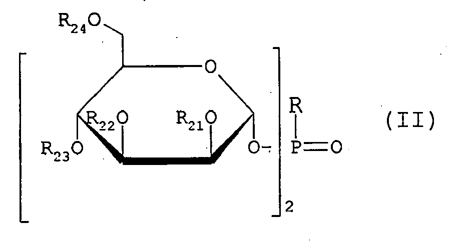 dans laquelle
   R₂₁, R₂₂, R₂₃ et R₂₄, identiques ou différents, représentent chacun l'atome d'hydrogène ou un groupe OH-protecteur, et
   R représente
   - un groupe OH,
   - un groupe alkoxy en C₁-C₂₀ (de préférence en C₁-C₅),
   - un groupe aryloxy en C₆-C₁₀ susceptible d'être substitué par un ou plusieurs groupes alkyle en C₁-C₅, alkoxy en C₁-C₅, halogéno, CF₃ et/ou nitro,
   - un groupe arylalkylèneoxy (notamment benzyloxy, phényléthyloxy, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
   - un groupe de structure:
      -O-CH(Q)-O-CO-alkyle, ou
      -O-CH (Q)-O-CO-O-alkyle
      où Q est H ou CH₃, et le reste alkyle est en C₁-C₅,
   - un groupe -O-CH₂-CH(OH)-CH₂OH, où les groupes OH peuvent être protégés,
   - un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
   - un groupe aminoacide de structure VIIa : 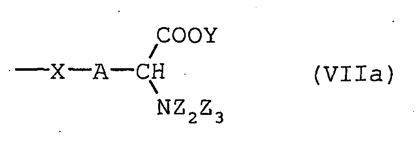 où
      X est -O-, -S- ou -NZ₁-,
      Y représente H ou un groupe alkyle en C₂-C₅,
      A est un groupe alkylène, phénylène ou phénylalkylène, (où chaque groupe alkylène est en C₁-C₅),
      Z₁ est H, un groupe alkyle en C₁-C₅ ou un groupe N-protecteur, et
      Z₂ et Z₃, identiques ou différents, représentent chacun H, un groupe alkyle en C₁-C₅, ou un groupe N-protecteur ;
   - un groupe aminoacide de structure VIIb : 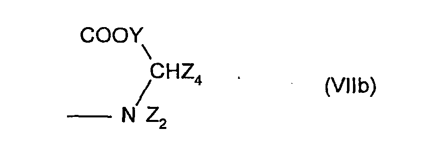 où
      Y représente H ou un groupe alkyle en C₂-C₅,
      Z₄ est un groupe alkylène, phénylène ou phénylalkylène, (où chaque groupe alkylène est en C₁-C₅) ou une chaine latérale des aminoacides naturels, protégée ou non par un groupement protecteur,
      Z₂ représente H, un groupe alkyle en C₁-C₅, ou un groupe N-protecteur;
(y) les tri(α-D-mannopyranosyl-1) phosphates de formule III' : 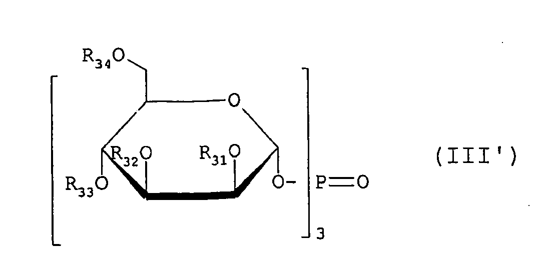 dans laquelle
   R₃₁, R_{32,} R₃₃ et R₃₄, identiques ou différents, représentent chacun un groupe OH-protecteur ayant au moins trois atomes de carbone ; et
(δ) leurs mélanges.

Enfin selon un autre aspect de l'invention,
l'on fournit un procédé pour préparer un composé de formule I, II ou III', ledit procédé étant caractérisé en ce qu'il comprend
(a) la réaction d'un 1-bromo-mannopyranose de formule (IVa) : 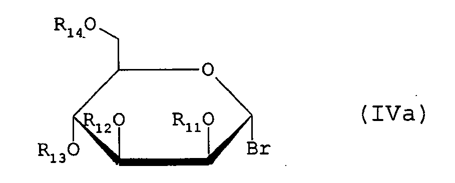 où R₁₁, R₁₂, R₁₃ et R₁₄ sont définis comme indiqué ci-dessus,
   avec un phosphate de monoargent de formule (Va) : 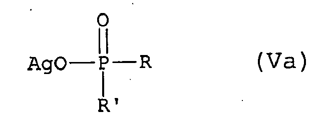 où R et R' sont définis comme indiqué ci-dessus,
   pour obtenir un composé mono(α-D-mannopyranosyl-1) phosphate de formule I;
(b) la réaction d'un 1-bromo-mannopyranose de formule (IVb) : 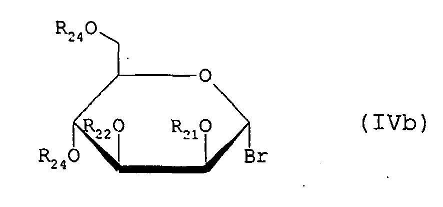 où R₂₁, R₂₂, R₂₃ et R₂₄ sont définis comme indiqué ci-dessus,
   avec un phosphate de diargent de formule (Vb) : 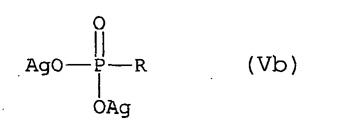 où R est défini comme indiqué ci-dessus,
   pour obtenir un composé di(α-D-mannopyranosyl-1) phosphate de formule II; ou
(c) la réaction d'un 1-bromo-mannopyranose de formule (IVc) : 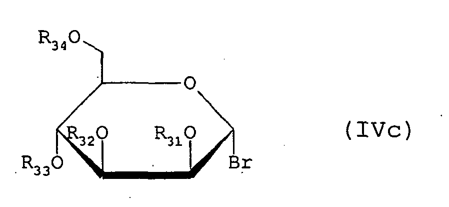 où R₃₁, R₃₂, R₃₃ et R₃₄, identiques ou différents, représentent chacun un groupe OH-protecteur qui est un groupe acyle en C₃-C₆,
   avec un phosphate de triargent de formule (Vc) : 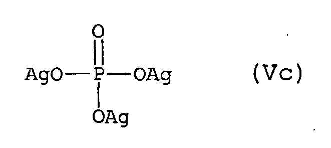 pour obtenir un composé tri(α-D-mannopyranosyl-1) phosphate de formule III'.

Dans ce procédé les éventuelles opérations usuelles de protection, déprotection puis reprotection des groupes hydroxyle du reste mannopyranosyl-1 et/ou du ou des groupes OH acides de PO-OH ont été omises par commodité.

En variante, le phosphate d'argent de formule Va, Vb ou, respectivement, Vc peut être remplacé par un phosphate de formule VIa, VIb ou, respectivement, VIc : 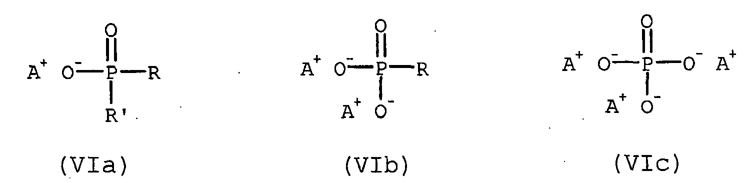 dans lesquelles R et R' sont définis comme indiqué ci-dessus, et A est H
ou R"₄N, R" étant H ou un groupe N-alkyle, cycloalkyle ou aromatique.

### Description détaillée de l'invention

Par groupe halogéno, on entend ici un atome d'halogène tel que F, Cl, Br ou I. Sur le plan de la synthèse, les halogènes préférés sont Cl et surtout Br. Sur le plan des propriétés pharmacologiques, les groupes halogéno préférés sur les groupes aromatiques sont F et Cl. Par ailleurs le groupe CF₃ est également un substituant présentant un intérêt certain au niveau des propriétés pharmacologiques.

Les groupes OH-protecteurs, qui interviennent selon l'invention pour protéger au moins un groupe hydroxyle du reste α-D-mannopyranosyle, sont des groupes usuellement utilisés dans le domaine des synthèses chimiques, notamment (i) dans celui des sucres et (ii) dans celui des peptides contenant des groupes latéraux hydroxylés. Ces groupes protecteurs sont en général éliminables pour restituer le ou les groupes hydroxyle concernés par la protection. Parmi les groupes OH-protecteurs qui conviennent ici, on peut notamment signaler les groupes acyle, en particulier en C₂-C₂₀ qui sont aliphatiques, aromatiques ou arylaliphatiques, notamment du type :
-CO-alkyle (où le groupe alkyle est en C₁-C₁₉ et de préférence en C₁-C₅),
-CO-aryle (où le groupe aryle est de préférence en C₆-C₁₀ et peut être substitué par un ou plusieurs groupes alkyle en C₁-C₅, alkoxy en C₁-C₅, halogéno, CF₃ et/ou nitro), et
-CO-alkylènearyle (où le groupe alkylène est avantageusement en C₁-C₅, et le groupe aryle est en C₆-C₁₀ et est susceptible d'être substitué par un ou plusieurs groupes alkyle en C₁-C₅, alkoxy en C₁-C₅, halogéno, CF₃ et/ou nitro).

De façon avantageuse, dans les formules I, II et III, le groupe OH-protecteur des fonctions OH en positions 2, 3, 4 et 6 du cycle mannosyle est essentiellement un groupe acyle aliphatique en C₂-C₆ (notamment COCH₃, COCH₂CH₃, COCH₂CH₂CH₃, COCH(CH₃)₂, CO(CH₂)₃CH₃, COC(CH₃)₃, COCH(CH₃)CH₂CH₃ ou COCH₂CH(CH₃)₂].

Dans la formule III', le groupe OH-protecteur des fonctions OH en positions 2, 3, 4 et 6 du cycle mannosyle est essentiellement (i) un groupe acyle aliphatique en C₃-C₆, notamment COCH₂CH₃, COCH₂CH₂CH₃, COCH(CH₃)₂, CO(CH₂)₃CH₃, COC(CH₃)₃, COCH(CH₃)CH₂CH₃ ou COCH₂CH(CH₃)₂.

Les groupes protecteurs du ou des groupes OH de la fonction acide PO-OH, qui interviennent selon l'invention, sont également classiques dans le domaine de la chimie organique. La protection concernée, qui est éventuellement temporaire, est principalement obtenue par estérification de la fonction acide PO-OH au moyen d'un composé présentant un groupe hydroxyle du type alcool (ou dérivé) ou phénol (ou dérivé). Des exemples de groupes protecteurs de chaque fonction acide PO-OH (i e. quand R et/ou R' = OH) sont donnés plus loin. Enfin les groupes N-protecteurs, qui interviennent ici dans la structure VII, sont bien connus dans la chimie des peptides.

Le groupe R des formules I et II (et il en est de même pour le groupe R' de la formule I) représente d'une manière générale :
- un groupe OH (notamment pour la synthèse d'autres mannopyranosyl-1 phosphates),
- un groupe OT, où T est un reste protecteur de la fonction acide PO-OH, ou
- un reste amino (en particulier quand R est un reste de structure VII, dans lequel le groupe X est -NZ₁-).

Ainsi le groupe R selon l'invention est :
(a) un groupe alkoxy en C₁-C₂₀ (i. e. T = alkyle en C₁-C₂₀), quand il s'agit d'un composé de formule II, de préférence en C₁-C₅,
(b) un groupe aryloxy en C₆-C₁₀ (i. e. T = aryle en C₆-C₁₀) qui est susceptible d'être substitué par un ou plusieurs groupes alkyle en C₁-C₅, alkoxy en C₁-C₅, halogéno, CF₃ et/ou nitro, notamment un groupe phénoxy, 4-méthoxyphénoxy, 3,4-dimethoxyphénoxy, 4-nitrophénoxy, 3-chlorophénoxy, 4-chlorophénoxy, 3,5-diméthylphénoxy, 3-trifluorométhylphénoxy, 1-naphtyloxy ou 2-naphtyloxy,
(c) un groupe (C₁-C₅)aryl-(C₆-C₁₀)alkylèneoxy, en particulier un groupe benzyloxy, phénéthyloxy, 1-naphtylméthyloxy ou 2-naphtylméthyloxy, où le reste aryle peut être substitué par un ou plusieurs groupes alkyle en C₁-C₅, alkoky en C₁-C₅, halogéno, CF₃ et/ou nitro,
(d) un groupe

   -O-CH(Q)-O-CO-(C₁-C₅)alkyle

   où Q est CH₃ dans la formule I, et H ou CH₃ dans la formule II, ou

   -O-CH(Q)-O-CO-O-(C₁-C₅)alkyle,

   où Q est H ou CH₃,
(e) un groupe -O-CH₂-CH(OH)-CH₂OH, où les groupes OH peuvent être protégés,
(f) un groupe OB, où B est un reste aliphatique C₂-C₂₁ éthyléniquement insaturé (qui peut comporter une ou plusieurs doubles liaisons C=C) à chaîne hydrocarbonée linéaire ou ramifiée, ou un reste cycloaliphatique en C₅-C₂₁, notamment un groupe -O-CH₂-CH=C(CH₃)₂ ou un groupe terpèneoxy dans lequel la portion terpène est cyclique ou acyclique, parmi les groupes R = terpèneoxy acycliques qui conviennent on peut citer de façon non limitative :
   le groupe farnésyloxy de structure (VIII) : 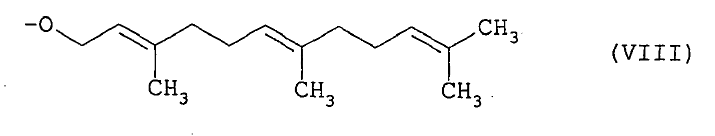 *[autre nomenclature : (3,7,11-triméthyl-2,6,10-dodécatriéne-1-yl)oxy],* et
   le groupe géranyloxy de structure (IX) : 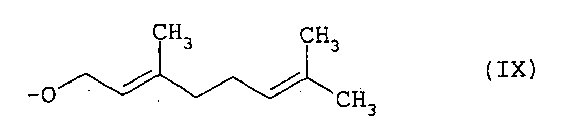 *[autre nomenclature : ((E)-3,7-diméthyl-2,6-octadiène-1-yl)oxy],*
(g) un groupe de structure VIIa : 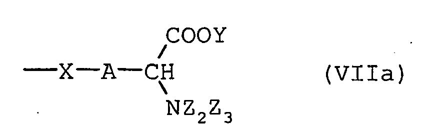 où X est -O-, -S- ou -NZ₁-, et, A, Y, Z₂ et Z₃ sont définis comme indiqué ci-dessus, et
(h) un groupe aminoacide de structure Vllb : 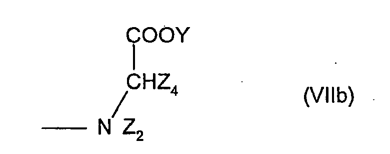 où
   Y représente H ou un groupe alkyle en C₂-C₅,
   Z₄ est un groupe alkylène, phénylène ou phénylalkylène, (où chaque groupe alkylène est en C₁-C₅) ou une chaine latérale des aminoacides naturels, protégée ou non par un groupement protecteur,
   Z₂ représente H, un groupe alkyle en C₁-C₅, ou un groupe N-protecteur.

De façon avantageuse, lesdites structures VIIa, VIIb pourront être élaborées à partir d'un aminoacide (avantageusement un aminoacide naturel) comportant une fonction amine ou hydroxyle latérale permettant la fixation de l'aminoacide basique ou hydroxylé sur l'atome de phosphore.

Parmi les aminoacides qui conviennent, on recommande tout particulièrement les aminoacides à chaîne latérale basique tels que la lysine, d'une part, et les aminoacides à chaîne latérale hydroxylée tels que la tyrosine, la sérine et la thréonine, d'autre part. On notera que la cystéine ou l'homocystéine tout comme la sérine ou l'homosérine sont des acides aminés qui conviennent également.

Les groupes R (ou R') préférés selon l'invention sont les suivants en ce qui concerne les composés de formule I ou II :
(α) un groupe phénoxy ou 1-naphtyloxy,
(β) un groupe benzyloxy ou 1-naphtylméthoxy,
(γ) un groupe -O-CH(Q)-O-CO-O-(C₁-C₅)alkyle, où Q est H ou CH₃, à l'exclusion de H pour le composé de formule I afin d'éviter la formation de formaldéhyde lors de la dégradation du composé,
(δ) un groupe -O-CH₂-CH(OH)-CH₂OH, où les groupes OH peuvent être protégés,
(ε) un groupe εLys, pTyr; βSer ou βThr, dont les structures (où les groupes NH₂ et COOH peuvent être protégés) sont les suivantes :
   εLys : -NH-(CH₂)₄-CH(NH₂)COOH,
   pTyr : -(p-O)-C₆H₄-CH₂-CH(NH₂)COOH,
   βSer: -O-CH₂-CH(NH₂)COOH, et
   βThr : -O-CH(CH₃)-CH(NH₂)COOH, et
(ζ) un groupe
   -NH-(CH₂)₃-CH(NH₂)COOH ou
   -NH-(CH₂)₂-CH(NH₂)COOH,
   où les fonctions NH₂ et COOH peuvent être protégées.

En ce qui concerne les composés de formule II, on peut également citer un autre groupe R préféré :
(η) -O-CH(Q)-O-CO-(C₁-C₅)alkyle, où Q est H ou CH₃.

Comme indiqué plus haut, l'invention concerne en particulier (a) en tant que médicaments, les mono(α-D-mannopyranosyl-1) phosphates de formule I, les di(α-D-mannopyranosyl-1) phosphates de formule II et les tri(α-D-mannopyranosyl-1) phosphates de formule III : 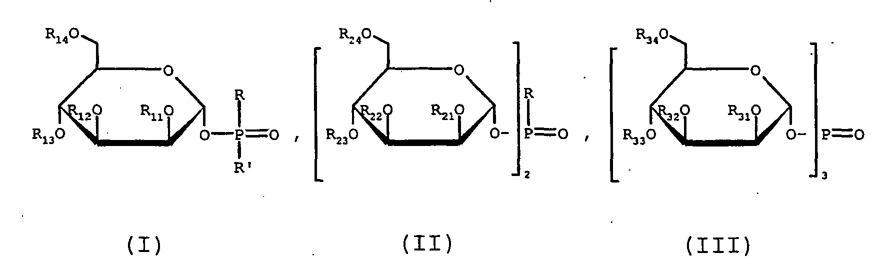 et (b) en tant que produits industriels nouveaux, les mono(α-D-mannopyranosyl-1) phosphates de formule I, les di(α-D-mannopyranosyl-1) phosphates de formule II et les tri(α-D-mannopyranosyl-1) phosphates de formule III' ci-dessus.

Les composés de formule I où R₁₁ = R₁₂ = R₁₃ = R₁₄ = H, et R = R' = OH, ceux de formule II où R₂₁ = R₂₂ = R₂₃ = R₂₄ = H et R = OH, et ceux de formule III où R₃₁ = R₃₂ = R₃₃ = R₃₄ = H sont (i) utiles sur le plan de la synthèse d'autres composés de l'invention et (ii) intéressants sur le plan pharmacologique.

Cependant, les composés de formules I, II et III, où tous les groupes OH sont protégés tant sur le cycle mannopyranosyle que sur l'atome de phosphore, interviennent de façon plus efficace que les précédents en tant que sources intracellulaires de Man-1 P : après avoir traversé la paroi cellulaire, ils sont déprotégés principalement par voie enzymatique pour fournir le Man-1 P requis dan le syndrome CDG-I et plus particulièrement dans le syndrome CDG-Ia.

De façon pratique, pour le traitement des patients souffrant du syndrome CDG-Ia, les composés selon l'invention qui sont les plus intéressants sont (dans un ordre d'intérêt décroissant) les suivants :
(1°) les di(2,3,4,6-tétra-O-acyl-α-D-mannopyranosyl-1) phosphates de formule II, dans lesquels le groupe OH-protecteur des groupes hydroxyle en positions 2, 3, 4 et 6 du cycle mannopyranosyle est tel que : R₂₁ = R₂₂ = R₂₃ = R₂₄ = acyle aliphatique en C₂-C₆ ;
(2°) les mono(2,3,4,6-tétra-O-acyl-α-D-mannopyranosyl-1) phosphates de formule I, dans lesquels R = R' et le groupe OH-protecteur des groupes hydroxyle en positions 2, 3, 4 et 6 du cycle mannopyranosyle est tel que : R₁₁ = R₁₂ = R₁₃ = R₁₄ = acyle aliphatique en C₂-C₆ ; puis
(3°) les tri(2,3,4,6-tétra-O-acyl-α-D-mannopyranosyl-1) phosphates de formule III, dans lesquels le groupe OH-protecteur des groupes hydroxyle en positions 2, 3, 4 et 6 du cycle mannopyranosyle est tel que : R₃₁ = R₃₂ = R₃₃ = R₃₄ = acyle aliphatique en C₂-C₆.

Dans les tableaux I, II et III, qui suivent, on a consigné un certain nombre de dérivés de (α-D-mannopyranosyl-1) phosphate typiques selon l'invention. Par commodité, les groupes R₁₁ à R₁₄, R₂₁ à R₂₄ et R₃₁ à R₃₄ y sont représentés par le même symbole R₁, d'une part, et les formules I, II et, respectivement, III sont désignées par Ia, IIa et, respectivement, IIIa, d'autre part.

Dans ces tableaux I, II et III, les abréviations utilisées sont les suivantes :
- Ac: acétyle,
- Bu: butyle [CH₂CH₂CH₂CH₃],
- iBu: isobutyle [CH₂CH(CH₃)₂],
- sBu: sec.-butyle [CH(CH₃)CH₂CH₃],
- tBu: tert.-butyle [C(CH₃)₃],
- Far: farnésyle [voir plus haut],
- Ger: géranyle [voir plus haut],
- εLys: reste ε-lysyle [NH-(CH₂)₄-CH(NH₂)COOH] dans lequel les fonctions NH₂ et COOH peuvent être protégées,
- Me: méthyle
- 1Napht: 1-naphtyle
- Pr: propyle [CH₂CH₂CH₃],
- iPr: isopropyle [CH(CH₃)₂],
- βSer: reste β-sérinyle [O-CH₂-CH(NH₂)COOH] dans lequel les fonctions NH₂ et COOH peuvent être protégées,
- βTyr: reste β-thréonyle [O-CH(CH₃)-CH(NH₂)COOH] dans lequel les fonctions NH₂ et COOH peuvent être protégées,
- pTyr: reste p-tyrosyle [(p-O-C₆H₄CH₂)-CH(NH₂)COOH] dans lequel les fonctions NH₂ et COOH peuvent être protégées,

**Tableau I**

| | | |
|---|---|---|
| 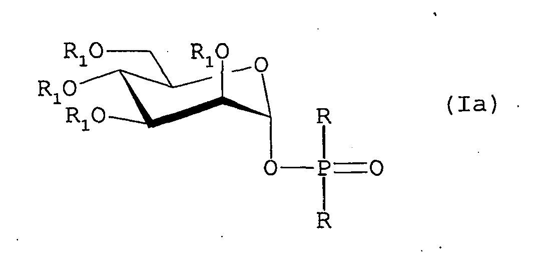 | | |

| Produit | R₁ | R |
|---|---|---|
| Ex. 20 | Ac | O-1Napht |
| Ex. 21 | Ac | O-C₆H₅ |
| Ex. 22 | Ac | O-CH₂C₆H₄(4-CF₃) |
| Ex. 23 | CO-tBu | O-CH₂C₆H₄(3,4-diMeO) |
| Ex. 24 | CO-sBu | O-CH(CH₃)-O-CO-CH₂CH₃ |
| Ex. 25 | CO-iPr | O-CH₂-O-CO-O-tBu |
| Ex. 26 | CO-Bu | O-CH₂-O-CO-O-iPr |
| Ex. 27 | Ac | εLys |
| Ex. 28 | Ac | pTyr |
| Ex. 29 | Ac | O-CH(CH₃)-O-CO-O-iPr |
| Ex. 30 | Ac | O-Far |
| Ex. 31 | Ac | O-Ger |
| Ex. 32 | Ac | O-CH₂-CH=C(CH₃)₂ |
| Ex. 33 | Ac | O-(CH₂)₂-CH=C(CH₃)₂ |

**Tableau II**

| | | |
|---|---|---|
| 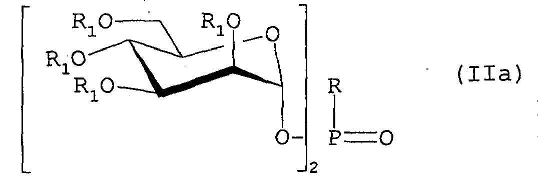 | | |

| Produit | R₁ | R |
|---|---|---|
| Ex. 1 | Ac | O-CH₂CH₃ |
| Ex. 2 | Ac | O-CH₂C₆H₅ |
| Ex. 3 | Ac | O-CH₂C₆H₄(4-NO₂) |
| Ex. 4 | CO-iPr | OH |
| Ex. 5 | Ac | O-C₆H₅ |
| Ex. 6 | CO-tBu | O-CH₂C₆H₄(4-OCH₃) |
| Ex. 7 | CO-tBu | O-CH₂-1Napht |
| Ex. 8 | CO-tBu | O-C₆H₅ |
| Ex. 9 | CO-tBu | O-CH₂-O-CO-tBu |
| Ex. 10 | CO-tBu | εLys |
| Ex. 11 | Ac | O-CH₂-1Napht |
| Ex. 12 | CO-iPr | O-CH₂C₆H₅ |
| Ex. 13 | CO-iPr | O-C₆H₅ |
| Ex. 14 | Ac | βTyr |
| Ex. 15 | CO-Pr | βSer |
| Ex. 16 | CO-iPr | pTyr |
| Ex. 17 | Ac | εLys |
| Ex. 18 | Ac | O-Far |
| Ex. 19 | Ac | O-Ger |
| Ex. 20 | Ac | O-CH₂-CH=C(CH₃)₂ |
| Ex. 21 | Ac | O-(CH₂)₂-CH=C(CH₃)₂ |

**Tableau III**

| | | | |
|---|---|---|---|
| 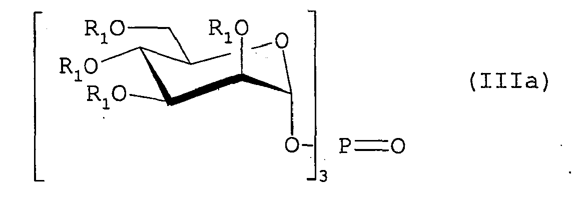 | | | |

| Produit | | R₁ | |
|---|---|---|---|
| Ex. 33 | | COCH₃ | |
| Ex. 34 | | CO-CH₂CH₃ | |
| Ex. 35 | | CO-Pr | |
| Ex. 36 | | CO-iPr | |
| Ex. 37 | | CO-tBu | |
| Ex. 38 | | CO-iBu | |
| Ex. 39 | | CO-Bu | |

Les composés de formule I, II ou III peuvent être préparés selon une méthode connue en soi par application de mécanismes réactionnels classiques. Le procédé de synthèse, que l'on, préconise selon l'invention met en oeuvre les réactions de substitution nucléophile (1), (2) ou (3) qui suivent :

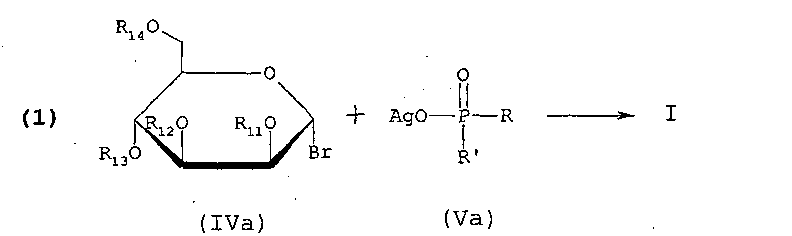

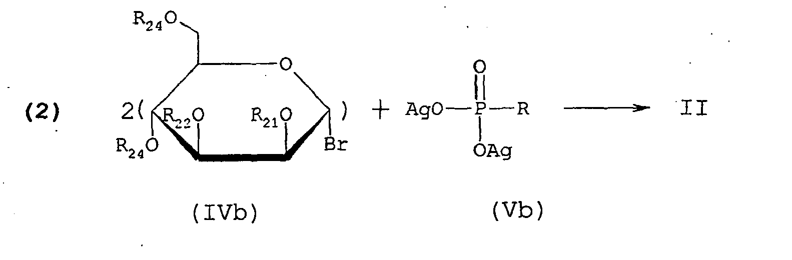

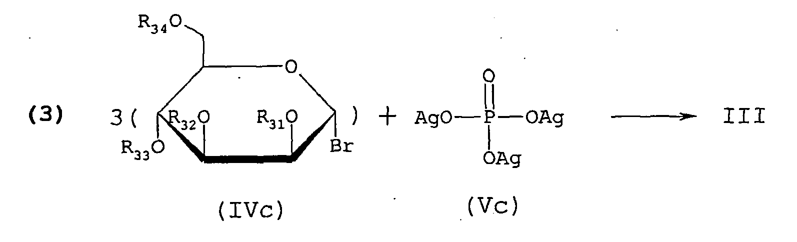

Chacune de ces réactions (1), (2) et (3) est mise en oeuvre à une température de 15 à 40 °C, de préférence à la température ambiante (RT = 15-25 °C) dans un solvant inerte approprié, en présence d'un tamis moléculaire. Un tel solvant est avantageusement le toluène. Le tamis moléculaire que l'on préconise de façon avantageuse est un tamis moléculaire de 4 Å (i. e. 0,4 µm).

Avantageusement, les opérations de protection, déprotection puis reprotection sont incluses dans les mécanismes réactionnels du procédé de préparation selon l'invention, à savoir :
(1 °) protection des groupes OH en positions 2, 3, 4 et 6 du reste mannopyranosyle lors de la synthèse des composé de formule IVa, IVb ou IVc, en amont des réactions (1), (2) ou (3), par acylation desdits groupes OH;
(2°) protection du ou des groupes acides R (ou R') = OH liés à l'atome de phosphore dans la formule Va, Vb ou Vc, par exemple au moyen d'un groupe R (ou R') = alkoxy (notamment éthyloxy), aryloxy (notamment phénoxy, 1-naphtyloxy ou 2-naphtyloxy) ou arylalkyloxy (notamment benzyloxy, 1-naphtylméthoxy ou 2-naphtylméthoxy), en amont de la réaction (1), (2) ou (3), par estérification du ou des groupes acides PO-OH avec un alcool ou dérivé ;
(3°) mise en oeuvre de la réaction (1), (2) ou (3) ;
(4°) le cas échéant, déprotection du groupe alkoxy R (ou R') différent de OH pour obtenir la ou les fonctions acides PO-OH ;
(5°) reprotection du ou des groupes acides R = OH, ainsi obtenus, par réaction d'estérification avec un alcool ou dérivé différent de celui de l'étape (2°) ou, respectivement, par réaction d'amidification avec une amine pour obtenir de nouveaux amides du type PO-NZ₁-A-CH(NH₂)COOH (où Z₁ est défini comme ci-dessus, et les fonctions NH₂ et COOH peuvent être protégées).

En pratique, il n'est pas nécessaire d'envisager la protection, la déprotection puis la reprotection des groupes OH en positions 2, 3, 4 et 6 du reste mannopyranosyle. Il suffit de partir d'un composé de formule IVa, IVb ou IVc comportant le reste acyle final souhaité en positions 2, 3, 4 et 6.

Pour ne pas compliquer les modalités de synthèse, on préfère plutôt avoir :
(1°) R₁₁ = R₁₂ = R₁₃ = R₁₄,
   R₂₁ = R₂₂ = R₂₃ = R₂₄, et
   R₃₁ = R₃₂ = R₃₃ = R₃₄, d'une part ; et
(2°) R = R', d'autre part.

En bref, l'on préconise selon l'invention un dérivé de (α-D-mannopyranosyl-1) phosphate qui est caractérisé en ce que :
- dans la formule I, R = R', d'une part, et le groupe OH-protecteur des groupes hydroxyle en positions 2, 3, 4 et 6 du cycle mannopyranosyle est tel que : R₁₁ = R₁₂ = R₁₃ = R₁₄ = acyle en C₂-C₆, d'autre part ;
- dans la formule II, le groupe OH-protecteur des groupes hydroxyle en positions 2, 3, 4 et 6 du cycle mannopyranosyle est tel que :
   R₂₁ = R₂₂ = R₂₃ = R₂₄ = acyle en C₂-C₆ ; et
- dans la formule III, le groupe OH-protecteur des groupes hydroxyle en positions 2, 3, 4 et 6 du cycle mannopyranosyle est tel que :
   R₃₁ = R₃₂ = R₃₃ = R₃₄ = acyle en C₂-C₆.

Les produits de l'invention peuvent être administrés chez les patients souffrant du syndrome CDG-la par voie orale qui est la voie la plus simple d'emploi et la plus appropriée chez la majorité des patients. Selon l'état des connaissances actuelles; une dose quotidienne délivrant environ 300 à 750 mg/kg de poids corporel de Man-1 P semble indiquée, eu égard aux doses de mannose utilisées dans le traitement *per os* du syndrome CDG-Ib dont le déficit enzymatique est juste en amont dans la séquence métabolique conduisant au mannose-6 phosphate, qui est le substrat de la PPM2.

Comme indiqué plus haut, les dérivés de (α-D-mannopyranosyl-1) phosphate, dans lesquels R (ou R') = OH, sont utilisables en tant qu'intermédiaires de synthèse des composés de formule I ou II où R est différent de OH.

D'autres avantages et caractéristiques de l'invention seront mieux compris à la lecture de la description qui va suivre (i) d'exemples de préparation et (ii) de résultats d'essais pharmacologiques. Bien entendu l'ensemble de ces éléments n'est nullement limitatif mais est fourni à titre d'illustration.

### Préparation I

Monoéthyl phosphate d'argent

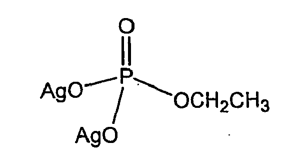

L'acide phosphoreux (5 g ; 61 mmol) est solubilisé dans une solution d'éthanol (53 mL) et de triéthylamine (30 mL ; 6,75 mmol). L'iode (23,2 g ; 91,4 mol) est ensuite ajouté en portions dans la solution refroidie à 5°C. Après 30 minutes d'agitation, le mélange est versé dans l'acétone (4.00 mL) à 0°C et un excès de cyclohexylamine (20 mL) est ajouté. Le précipité formé est filtré, lavé à l'acétone et recristallisé dans l'éthanol à chaud. Le rendement en éthyl phosphate de dicyclohexylammonium est de 80%.

Une solution d'éthyl phosphate de dicyclohexylammonium dans l'eau distillée est échangée sur une colonne (5 x 3,5 cm) de Dowex 50W x 8 - 100 sous forme Na+ . La résine est lavée avec 5 volumes d'eau pure par rapport à la résine. Après concentration des éluats, le produit sous forme de sel de sodium (1,684 mg; 1 mmol) est repris dans l'eau (2 mL) et une solution de AgNO₃ (378,8 mg ; 2,23 mmol) dans l'eau (2 mL) est ajoutée. Le mélange est agité à l'obscurité, à température ambiante. Le précipité formé est ensuite filtré, rincé successivement à l'eau à 0°C, l'éthanol et l'éther, puis séché. Le monoéthyl phosphate d'argent, ainsi obtenu, est conservé à -20°C.

### Préparation II (Ex. 1)

### Ethyle di[2,3,4,6-tétra-O-acétyl-α-D-mannopyranosyl-1] phosphate [Autre nomenclature : phosphate de di(2, 3, 4, 6-tétra-O-acétyl-a D-mannopyranosyl-1) et d'éthyle]

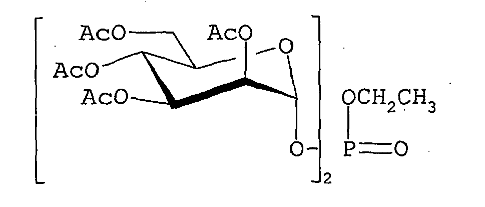

Le monoéthyl phosphate d'argent (333 mg ; 0,9 mmol), obtenu selon le procédé de la Préparation I, est agité, en suspension dans le toluène anhydre (3 ml) avec un tamis moléculaire 4Å activé, pendant 30 minutes à température de 15-20 °C. Une solution de 1-bromo-(2,3,4,6-tétra-O-acétyl)-α-D-mannopyranose (732,3 mg ; 1,78 mmol) dans le toluène (2 mL) est ajoutée et le mélange est agité à température ambiante pendant 6 h. Après filtration sur célite et évaporation du toluène, le mélange est purifié par chromatographie sur colonne de silice (éluant : cyclohexane/acétate d'éthyle : 5/5 v/v avec 3 %o de triéthylamine). Le produit attendu est obtenu pur avec un rendement de 65% (455 mg ; 0,59 mmol).
[α]_{D}²⁰ = + 38 (c 1,0 ; CH₂Cl₂)
***RMN* ¹H** (CDCl₃, 250 MHz): δ 5,72 (d, 2 H, *J*₁,₂ = 5,5 Hz, H-1), 5,32-5,39 (m, 6 H, H-2, H-3, H-4), 4,12-4,42 (m, 8 H, H-5, 2H-6, C*H*₂-CH₃), 2,02 ; 2,08 ; 2,12 ; 2,19 (s, 24 H, 4 acétyles), 1,43 (t, 3 H, *J* = 7,25 Hz, CH₂-C*H*₃)
***RMN* ¹³C** (CDCl₃, 63 MHz) : δ 170,6 ; 169,9 ; 169,8 ; 169,6 (CO acétates), 95,6; 95,5 (2 d, *J*_{C1-P} = 6,3 Hz, C-1^{A}, C-1^{B}), 70,8 ; 70,6 (C-5^{A}, C-5^{B}), 68,9; 68,7 (2 d, *J*_{C2-P} = 3 Hz, C-2^{A}, C-2^{B}), 68,2 (C-4^{A}, C-4^{B}), 65,5 (d, *J*_{C-P} = 6,3Hz, CH₂CH₃), 65,4; 65,2 (C-3^{A}, C-3^{B}), 62,0; 61,8 (C-6^{A}, C-6^{B}), 20,7 (CH₃ acétates), 16,2 (d, *J*_{C-P} = 6,2 Hz, CH₃CH₂)
***RMN* ³¹P** (CDCl_{3,} 250 MHz) : δ -15 ppm
***SMHR Electrospray*** (mode positif) : calculé pour C₃₀H₄₇O₂₂NP [M + NH4]⁺ : 804,2327 ; trouvé : 804,2329.

### Préparation III (Ex. 12)

### Benzyle di(2,3,4,6-tétra-O-iso-butyryl-(α-D-mannopyranosyl-1) phosphate [Autre nomenclature : phosphate de di(2,3,4,6-tétra-O-iso-butyryl-α-D-mannopyranosyl-1) et de benzyle]

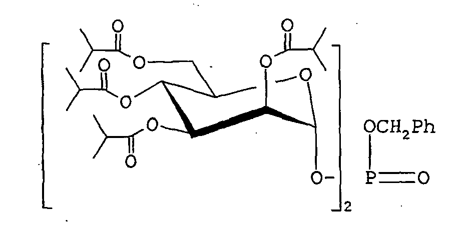

Le benzylphosphate de diargent (148,08 mg ; 0,368 mmol) en suspension dans le toluène anhydre (3mL) avec un tamis moléculaire 4Å activé (0,5 g) est agité pendant 30 min à température ambiante. Ensuite, une solution de 1-bromo-(2,3,4,6-tétra-O-iso-butyryl)-α-D-mannopyranose (350 mg ; 0,67 mmol) est ajoutée sous argon et le mélange est agité à température ambiante pendant une nuit. Après filtration et évaporation des solvants, le résidu repris dans du CH₂Cl₂ est purifié sur une colonne de Sephadex LH-20 et élué avec un mélange CH₂Cl₂/MeOH : 7/3 v/v. Le produit attendu, est isolé avec un rendement de 80% (280 mg ; 0,26 mmol).
[α]_{D}²⁰ = + 44 (c 1.0, CH₂Cl₂)
***RMN* ¹H** (CDCl_{3;} 250 MHz): δ 5,71 (d, 1 H, *J*_{H1a-P} = 7,5 Hz, H-1^{A}), 5,69 (dd, 1 H, *J*_{H1B-P} = 5,6 Hz, *J*_{H1B-2} = 1,5 Hz, H-1^{B}), 5,52 ; 5,47 (2 dd, 2 H, *J*_{H4-5} = 10 Hz, *J*4-3 = 10 Hz, H-4), 5,42-5,34 (m, 2H, H-3), 5,29 (t, 2 H, *J*_{H2-3} = 2,5 Hz, H-2), 5,19 (d, 2 H, *J* = 8,8 Hz, CH₂ benzyle), 4,40 (dd, 1 H, *J*_{H6a}-₅ₐ = 2,5 Hz, *J*_{H6a-6b} = 12,5 Hz, H-6^{A}), 4,27 (d, 1 H, *J*_{H5b-4} = 10 Hz, H-5^{B}), 4,15-4,00 (m, 3 H, H-5^{A}, H-6^{B}, H-6'^{A}), 3,90 (d, 1 H, *J*_{H6'a-6'b} = 10 Hz, H-6'^{B}), 2,64-2,36 (m, 8 H, C*H*(CH₃)₂), 1,26 -1,00 (m, 48 H, CH(C*H*₃)₂).
***RMN* ¹³C** (CDCl₃, 63 MHz): δ 176,7; 176,6; 175,8; 175,6 (CO isobutyrates), 135,1 (d, *J*_{C-P} = 6,8 Hz, C_{quat} aromatique), 129,4; 129,2; 128,6 (CH aromatiques), 96,0; 96,3 (C-1^{A}, C-1^{B}), 71,4 ; 71,2 (C-5^{A}, C-5^{B}), 70,9 (d, *J*_{C-P} = 6,3 Hz, CH₂Ph), 68,7 (C-2, C-3), 64,5 (C-4), 61,2 (C-6), 34,2 (CH(CH₃)₂), 19,3 ; 19,1 (CH(CH₃)₂)
**RMN ³¹P** (CDCl₃, 250 MHz) : δ -15,5 ppm
***SMHR Electrospray*** (mode positif) : calculé pour C₅₁ H₇₇O₂₂PNa [M+Na]⁺ : 1095,4542 ; Trouvé : 1095,4521

### Préparation IV (Ex. 4)

### Di(2,3,4,6-tétra-O-isobutyryl-α-D-mannopyranosyl-1) hydrogénophosphate [Autre nomenclature : hydrogénophosphate de di(2,3,4,6-tétra-O-isobutyryl-α-D-mannopyranosyl-1)]

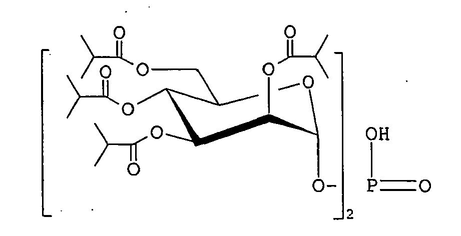

Le benzyle di(2,3,4,6-tétra-O-isobutyryl-α-D-mannopyranosyl-1) phosphate (48 mg ; 44,8 µmol), obtenu selon le procédé de la Préparation III, est agité dans le méthanol (2 mL) en présence de Pd/C 10% (20 mg) sous atmosphère de H₂ pendant 5h. Le mélange réactionnel est ensuite filtré et les solvants évaporés. Le produit débenzylé attendu est obtenu avec un rendement de 93% (41 mg ; 41,7 µmol)
[α]_{D}²⁰ = + 9 (c 1,0 ; CH₂Cl₂).
***RMN* ¹H** (CDCl₃, 250 MHz) : δ 5,58 (d, 2 H, H-1, *J*_{H1-P} = 5 Hz), 5,20-5,50 (m, 6 H, H-2, H-3, H-4), 4,32 (d/, 4 H, *J*_{H6-6'} = 10 Hz, H-6, H-6'), 4,20 (m, 2H, H-5), 2,69-2,41 (m, 8H, C*H*(CH₃)₂), 1,27-1,00 (m, 48 H, CH(C*H*₃)₂)
***RMN* ¹³C** (CDCl₃, 63 MHz): δ 175,7; 176,4; 177,0 (CO isobutyrates), 94,4 (C-1), 70,0-69,0 (C-2, C-3, C-5), 66,0 (C-4), 62,0 (C-6), 34,2 (CH(CH₃)₂), 18,8; 19,2; 19,4 (CH(CH₃)₂)
***RMN* ³¹P** (CDCl₃, 250 MHz) : δ -20 ppm
***MSHR Electrospray*** (mode positif) : calculé pour C₄₄H₇₀O₂₂PNa₂ [M-H+2Na]⁺ : 1027,3892 ; Trouvé : 1027,3905

### Essais pharmacologiques

Les composés de formule I, II et III ont été testés en tant que prodrogues (i. e. en tant que sources intracellulaires de Man-1 P), d'une part, pour évaluer leur toxicité et, d'autre part, pour apprécier leur capacité à inhiber l'incorporation de 2[³H]mannose au sein des glycoconjugués cellulaires. En effet, s'ils peuvent générer du Man-1 P dans les cellules, celui-ci sera en compétition avec le 2[³H]mannose-1 phosphate pour entrer dans la voie de biosynthèse des glycoprotéines (voir Eklund, E.A., *et al.* précité).

Les premiers résultats obtenus sont consignés dans le Tableau IV, qui suit. Les produits ont été testés (5 essais par produit et par dose) sur des lymphoblastes de malades CDG-la et leurs activités ("radioactivité", i. e. inhibition, par compétition, de la fixation de 2[³H]mannose-1 phosphate ; et "toxicité" cellulaire) ont été appréciées en pourcentage par rapport aux témoins (10 essais).

Ces résultats montrent que les meilleurs produits de l'art antérieur CP1, CP3 et CP6 (à la dose de 500 µM)) inhibent à au moins 90%, l'incorporation de 2[³H]mannose dans les glycoprotéines. Néanmoins, cet effet s'accompagne d'un doublement au moins de la mortalité cellulaire. Tous ces effets ont été observés sur des lymphoblastes issus de sujets sains et de patients CDG-la. En revanche, pour les produits des exemples 5, 11, 12 et 13 (utilisés à la dose de 500 µM), les premiers résultats sont très favorables. En effet, même si Ex. 5, Ex. 11, Ex. 12 et Ex. 13 sont moins efficaces que CP1, CP3 et CP6 à inhiber l'incorporation de mannose radioactif dans les glycoprotéines, ils sont toutefois beaucoup moins toxiques qu'eux. Le produit de l'exemple 4, qui est un produit acide de formule II (où R = OH), est moins toxique et thérapeutiquement plus intéressant que CP1, CP3 et CP6 ; cependant il semble thérapeutiquement moins efficace que Ex. 5, Ex. 11, Ex. 12 et Ex. 13.

**Tableau IV**

| Produits | Radioactivité | Toxicité |
|---|---|---|
| CP1 | 92 % | 210 % |
| CP3 | 90 % | 205 % |
| CP6 | 91 % | 201 % |
| Ex. 4* | 100 % | 100 % |
| Ex. 5 | 80 % | 120 % |
| Ex. 11 | 40 % | 115 % |
| Ex. 12 | 25 % | 112 % |
| Ex. 13 | 21 % | 110 % |
| Note | | |
| (*) : composé acide (R = OH) | | |

## Revendications

1. Composition pour une utilisation comme médicament, **caractérisée en ce qu'**elle renferme, en association avec un excipient physiologiquement acceptable, une substance active choisie parmi l'ensemble constitué par :
(α) les mono(α-D-mannopyranosyl-1) phosphates de formule I : 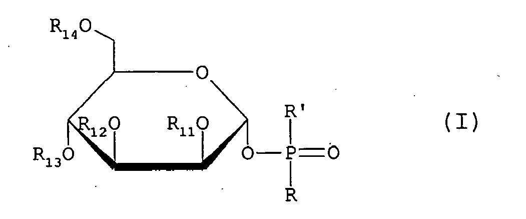 dans laquelle
R₁₁, R₁₂, R₁₃ et R₁₄, identiques ou différents, représentent chacun l'atome d'hydrogène ou un groupe OH-protecteur,
R et R', identiques ou différents, représentent chacun
• un groupe aryloxy en C₆-C₁₀ susceptible d'être substitué par un ou plusieurs groupes alkyle en C₁-C₅, alkoxy en C₁-C₅, halogéno, CF₃ et/ou nitro,
• un groupe arylalkylèneoxy, où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
• un groupe de structure :
-O-CH(CH₃)-O-CO-alkyle, ou
-O-CH(CH₃)-O-CO-O-alkyle
où le reste alkyle est en C₁-C₅,
• un groupe -O-CH₂-CH(OH)-CH₂OH, où les groupes OH peuvent être protégés,
• un reste OB, où B est un reste aliphatique en C₂-C₂₁ éthyléniquement insaturé à chaîne hydrocarbonée linéaire ou ramifiée, ou un reste cycloaliphatique en C₅-C₂₁,
• un groupe aminoacide de structure VIIa : 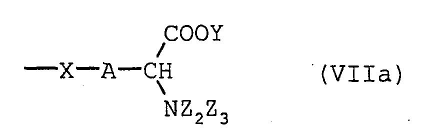 où
X est -O-, -S- ou -NZ₁-,
Y représente H ou un groupe alkyle en C₂-C₅,
A est un groupe alkylène, phénylène ou phénylalkylène, (où chaque groupe alkylène est en C₁-C₅),
Z₁ est H, un groupe alkyle en C₁-C₅ ou un groupe N-protecteur,
Z₂ et Z₃, identiques ou différents, représentent chacun H, un groupe alkyle en C₁-C₅, ou un groupe N-protecteur ;
• un groupe aminoacide de structure VIIb : 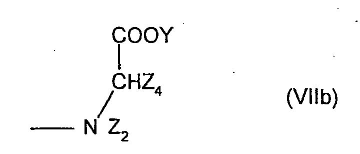 où
Y représente H ou un groupe alkyle en C₂-C₅,
Z₄ est un groupe alkylène, phénylène ou phénylalkylène, (où chaque groupe alkylène est en C₁-C₅),
Z₂ représente H, un groupe alkyle en C₁-C₅, ou un groupe N-protecteur ;
(β) les di(α-D-mannopyranosyl-1) phosphates de formule II : 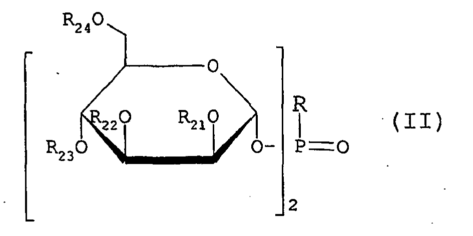 dans laquelle
R₂₁, R₂₂, R₂₃ et R₂₄, identiques ou différents, représentent chacun l'atome d'hydrogène ou un groupe OH-protecteur, et
R représente
• un groupe OH,
• un groupe alkoxy en C₁-C₂₀,
• un groupe aryloxy en C₆-C₁₀ susceptible d'être substitué par un ou plusieurs groupes alkyle en C₁-C₅, alkoxy en C₁-C₅, • halogéno, CF₃ et/ou nitro,
• un groupe arylalkylèneoxy, où le reste alkylène est en C₁-C₅, et le reste aryle, qui est en C₆-C₁₀, est susceptible d'être substitué
• par un ou plusieurs groupes alkyle en C₁-C₅, alkoxy en C₁-C₅, halogéno, CF₃ et/ou nitro,
• un groupe de structure:
-O-CH(Q)-O-CO-alkyle, ou
-O-CH(Q)-O-CO-O-alkyle
où Q est H ou CH₃, et le reste alkyle est en C₁-C₅,
• un groupe -O-CH₂-CH(OH)-CH₂OH, où les groupes OH peuvent être protégés,
• un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
• un groupe aminoacide de structure VIIa : 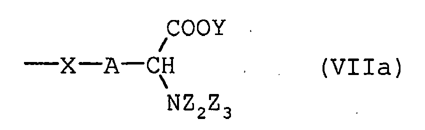 où
X est -O-, -S- ou -NZ₁-,
Y représente H ou un groupe alkyle en C₂-C₅,
A est un groupe alkylène, phénylène ou phénylalkylène, (où chaque groupe alkylène est en C₁-C₅),
Z₁ est H; un groupe alkyle en C₁-C₅ ou un groupe N-protecteur, et
Z₂ et Z₃, identiques ou différents, représentent chacun H, un groupe alkyle en C₁-C₅, ou un groupe N-protecteur ;
• un groupe aminoacide de structure VIIb : 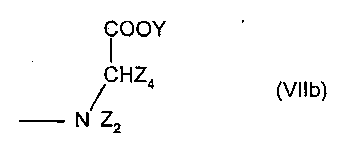 où
Y représente H ou un groupe alkyle en C₂-C₅,
Z₄ est un groupe alkylène; phénylène ou phénylalkylène, (où chaque groupe alkylène est en C₁-C₅),
Z2 représente H, un groupe alkyle en C₁-C₅, ou un groupe N-protecteur;
(γ) les tri(α-D-mannopyranosyl-1) phosphates de formule III : 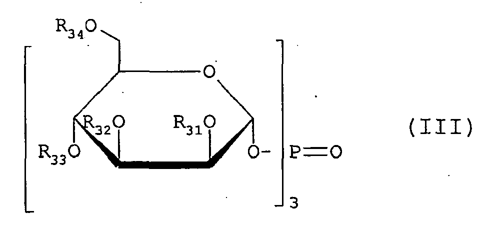 dans laquelle ,
R₃₁, R₃₂, R₃₃ et R₃₄, identiques ou différents, représentent chacun un atome d'hydrogène ou groupe OH-protecteur ; et
(δ) leurs mélanges.

2. Composition suivant la revendication 1, pour une utilisation comme médicament vis-à-vis du syndrome CDG-I.

3. Composition suivant la revendication 2, pour une utilisation comme médicament vis-à-vis du syndrome CDG-Ia.

4. Composition suivant l'une quelconque des revendications 1 à 3, **caractérisée en ce que** ledit groupe OH-protecteur des groupes hydroxyle en positions 2, 3, 4 et 5 du cycle mannopyranosyle est un groupe acyle.

5. Composition suivant l'une quelconque des revendications 1 à 3, **caractérisée en ce que**, dans les formules I, II et III, le groupe OH-protecteur des fonctions OH en positions 2, 3, 4 et 6 du cycle mannosyle est un groupe acyle aliphatique en C₂-C₆.

6. Utilisation d'un dérivé de (mannosyl-1) phosphate, ladite utilisation étant **caractérisée en ce que** l'on fait appel à une substance intervenant en tant que source intracellulaire de Man-1 P, qui est choisie parmi l'ensemble constitué par les composés
(α) mono(α-D-mannopyranosyl-1) phosphates de formule I,
(β) di(α-D-mannopyranosyl-1) phosphates de formule II,
(γ) tri(α-D-mannopyranosyl-1) phosphates de formule III, et
(δ) leurs mélanges,
selon l'une quelconque des revendications 1 à 3,
pour la préparation d'un médicament destiné à un usage en thérapeutique vis-à-vis du syndrome CDG-1, et en particulier vis-à-vis du syndrome CDG-la.

7. Dérivé de mannosyl-1 phosphate, pour une utilisation comme médicament, **caractérisé en ce qu'**il est choisi parmi l'ensemble constitué par :
(α) les mono(α-D-mannopyranosyl-1) phosphates de formule I : 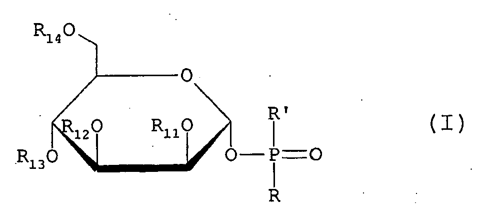 dans laquelle
R₁₁, R₁₂, R₁₃ et R₁₄, identiques ou différents, représentent chacun l'atome d'hydrogène ou un groupe OH-protecteur,
R et R', identiques ou différents, représentent chacun
• un groupe aryloxy en C₆-C₁₀ (notamment phénoxy, 1-naphtyloxy ou 2-naphtyloxy) susceptible d'être substitué par un ou plusieurs groupes alkyle en C₁-C₅, alkoxy en C₁-C₅, halogéno, CF₃ et/ou nitro,
• un groupe arylalkylèneoxy (notamment OCH₂CH₂C₆H₅, OCH₂C₆H₅, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
• un groupe de structure :
-O-CH(CH₃)-O-CO-alkyle, ou
-O-CH(CH₃)-O-CO-O-alkyle
où le reste alkyle est en C₁-C₅,
• un groupe -O-CH₂-CH(OH)-CH₂OH, où les groupes OH peuvent être protégés,
• un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
• un groupe aminoacide de structure VIIa: 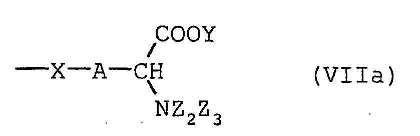 où
X est -O-, -S- ou -NZ₁-,
Y représente H ou un groupe alkyle en C₂-C₅,
A est un groupe alkylène, phénylène ou phénylalkylène, (où chaque groupe alkylène est en C₁-C₅),
Z₁ est H, un groupe alkyle en C₁-C₅ ou un groupe N-protecteur, et
Z₂ et Z₃, identiques ou différents, représentent chacun H, un groupe alkyle en C₁-C₅, ou un groupe N-protecteur ;
• un groupe aminoacide de structure VIIb : 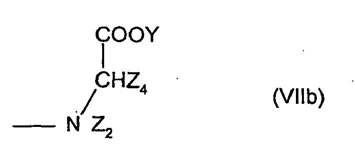 où
Y représente H ou un groupe alkyle en C₂-C₅,
Z₄ est un groupe alkylène, phénylène ou phénylalkylène, (où chaque groupe alkylène est en C₁-C₅),
Z₂ représente H, un groupe alkyle en C₁-C₅, ou un groupe N-protecteur ;
(β) les di(α-D-mannopyranosyl-1) phosphates de formule II : 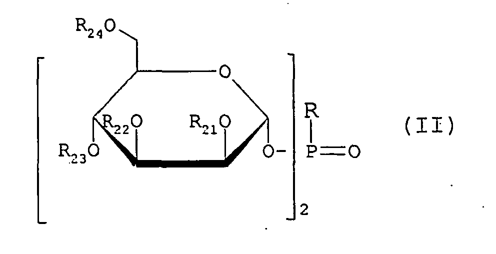 dans laquelle
R₂₁, R₂₂, R₂₃ et R₂₄, identiques ou différents, représentent chacun l'atome d'hydrogène ou un groupe OH-protecteur, et
R représente
• un groupe OH,
• un groupe alkoxy en C₁-C₂₀ (de préférence en C₁-C₅),
• un groupe aryloxy en C₆-C₁₀ susceptible d'être substitué par un ou plusieurs groupes alkyle en C₁-C₅, alkoxy en C₁-C₅, halogéno, CF₃ et/ou nitro,
• un groupe arylalkylèneoxy (notamment benzyloxy, phényléthyloxy, 1-naphtylméthyloxy ou 2-naphtylméthyloxy), où le reste alkylène est en C₁-C₅, et le reste aryle, qui est en C₆-C₁₀, est susceptible d'être substitué par un ou plusieurs groupes alkyle en C₁-C₅, alkoxy en C₁-C₅, halogéno, CF₃ et/ou nitro,
• un groupe de structure:
-O-CH(Q)-O-CO-alkyle, ou
-O-CH(Q)-O-CO-O-alkyle
où Q est H ou CH₃, et le reste alkyle est en C₁-C₅,
• un groupe -O-CH₂-CH(OH)-CH₂OH, où les groupes OH peuvent être protégés,
• un reste OB, où B est un reste aliphatique en C₂-C₂₁ éthyléniquement insaturé à chaîne hydrocarbonée linéaire ou ramifiée, ou un reste cycloaliphatique en C₅-C₂₁, ou
• un groupe aminoacide de structure VII : 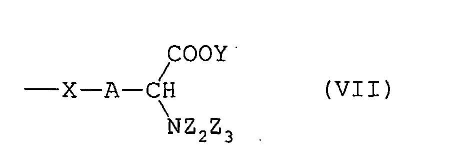 où
X est -O-, -S- ou -NZ₁-,
Y représente H ou un groupe alkyle en C₂-C₅,
A est un groupe alkylène, phénylène ou phénylalkylène, (où chaque groupe alkylène est en C₁-C₅),
Z₁ est H, un groupe alkyle en C₁-C₅ ou un groupe N-protecteur, et
Z₂ et Z₃, identiques ou différents, représentent chacun H, un groupe alkyle en C₁-C₅, ou un groupe N-protecteur ;
• un groupe aminoacide de structure VIIb: 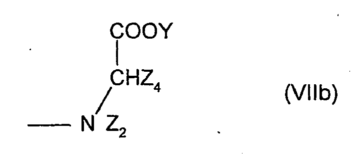 où
Y représente H ou un groupe alkyle en C₂-C₅,
Z₄ est un groupe alkylène, phénylène ou phénylalkylène, (où chaque groupe alkylène est en C₁-C₅),
Z₂ représente H, un groupe alkylé en C₁-C₅, ou un groupe N-protecteur ;
(γ) les tri(α-D-mannopyranosyl-1) phosphates de formule III' : 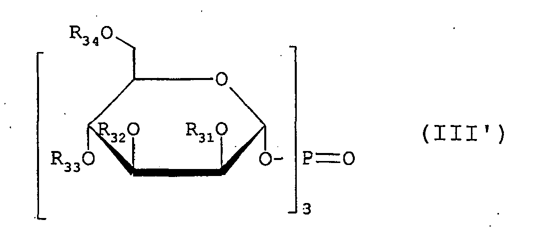 dans laquelle
R₃₁, R₃₂, R₃₃ et R₃₄, identiques ou différents, représentent chacun un groupe OH-protecteur ayant au moins trois atomes de carbone ; et
(δ) leurs mélanges.

8. Dérivé de (α-D-mannosyl-1) phosphate suivant la revendication 7, pour une utilisation comme médicament vis-à-vis du syndrome CDG-I.

9. Dérivé de (α-D-mannosyl-1) phosphate suivant la revendication 8, pour une utilisation comme médicament vis-à-vis du syndrome CDG-Ia.

10. Dérivé de (α-D-mannosyl-1) phosphate suivant l'une quelconque des revendications 7 à 9, **caractérisé en ce que**, dans les formules I est II, le groupe OH-protecteur des fonctions OH en positions 2, 3, 4 et 6 du cycle mannosyle est un groupe acyle aliphatique en C₂-C₆, et **en ce que**, dans la formule III', le groupe OH-protecteur des fonctions OH en positions 2, 3, 4 et 6 du cycle mannosyle est un groupe acyle aliphatique en C₃-C₆.

11. Dérivé de (α-D-mannosyl-1) phosphate suivant l'une quelconque des revendications 7 à 9, **caractérisé en ce que**, le groupe R (ou R') est un groupe OB où B est un reste aliphatique en C₂-C₂₁ éthyléniquement insaturé, pouvant comporter une ou plusieurs doubles liaisons C=C, à chaîne hydrocarbonée linéaire ou ramifiée, ou un reste cycloaliphatique en C₅-C₂₁, OB étant notamment un groupe -O-CH₂-CH=C(CH₃)₂ , O-(CH₂)₂-CH=C(CH₃)₂ ou un groupe terpèneoxy.

12. Dérivé de (α-D-mannosyl-1) phosphate suivant l'une quelconque des revendications 7 à 9 ou 11, **caractérisé en ce que**, le groupe R (ou R') est un groupe OB = terpèneoxy, dans lequel la portion terpène est cyclique ou acyclique, OB étant notamment un groupe farnésyloxy ou géranyloxy.

13. Dérivé de (α-D-mannosyl-1) phosphate suivant l'une quelconque des revendications 7 à 9, **caractérisé en ce que**, le groupe R (ou R') est un groupe de structure VII provenant d'un aminoacide comportant une fonction amine ou hydroxyle latérale.

14. Dérivé de (α-D-mannosyl-1) phosphate suivant l'une quelconque des revendications 7 à 9, **caractérisé en ce que** le groupe R (ou R') représente dans la formule I ou II :
(α) un groupe phénoxy ou 1-naphtyloxy,
(β) un groupe benzyloxy ou 1-naphtylméthoxy,
(γ) un groupe -O-CH(Q)-O-CO-O-(C₁-C₅)alkyle, où Q est H ou CH₃,
(δ) un groupe -O-CH₂-CH(OH)-CH₂OH, où les groupes OH peuvent être protégés,
(ε) un groupe εlys, pTyr; βSer ou βTyr, dont les structures (où les groupes NH₂ et COOH peuvent être protégés) sont les suivantes :
εLys : -NH-(CH₂)₄-CH(NH₂)COOH,
pTyr : _{:}(p-O)-C₆H₄-CH₂-CH(NH₂)COOH,
βSer: -O-CH₂-CH(NH₂)COOH, et
βTyr: -O-CH(CH₃)-CH(NH₂)COOH, ou
(ζ) un groupe
-NH-(CH₂)₃-CH(NH₂)COOH ou
-NH-(CH₂)₂-CH(NH₂)COOH,
où les fonctions NH₂ et COOH peuvent être protégées.
R pouvant également représenter dans la formule II :
(η) un groupe -O-CH(Q)-O-CO-(C₁-C₅)alkyle, où Q est H ou CH₃.

15. Procédé pour préparer un composé de formule I, II ou III' selon l'une quelconque des revendications 7 à 9, ledit procédé étant **caractérisé en ce qu'**il comprend
(a) la réaction d'un 1-bromo-mannopyranose de formule (IVa) : 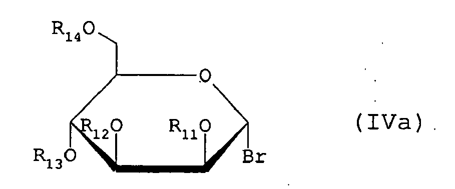 où R₁₁, R₁₂, R₁₃ et R₁₄ sont définis comme indiqué ci-dessus,
avec un phosphate de monoargent de formule (Va) : 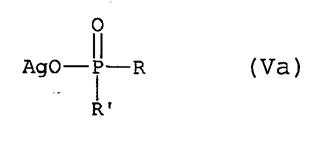 où R et R' sont définis comme indiqué ci-dessus,
pour obtenir un composé mono(α-D-mannopyranosyl-1) phosphate de formule 1 ;
(b) la réaction d'un 1-bromo-mannopyranose de formule (IVb) : 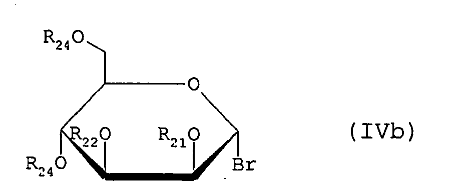 où R₂₁, R₂₂, R₂₃ et R₂₄ sont définis comme indiqué ci-dessus,
avec un phosphate de diargent de formule (Vb) : 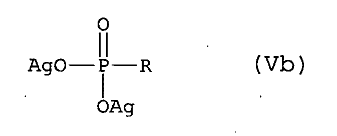 où R est défini comme indiqué ci-dessus,
pour obtenir un composé di(α-D-mannopyranosyl-1) phosphate de formule II; ou
(c) la réaction d'un 1-bromo-mannopyranose de formule (IVc) : 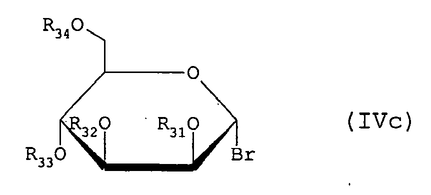 où R₃₁, R₃₂, R₃₃ et R₃₄, identiques ou différents, représentent chacun un groupe OH-protecteur qui est un groupe acyle en C₃-C₆,
avec un phosphate de triargent de formule (Vc) : 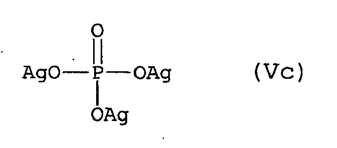 pour obtenir un composé tri(α-D-mannopyranosyl-1) phosphate de formule III'.

16. Procédé suivant la revendication 15, **caractérisé en ce que** la réaction de IVa avec Va, la réaction de IVb avec Vb ou la réaction de IVc avec Vc est mise en oeuvre à une température de 15 à 40 °C, de préférence à la température ambiante (15-25 °C), avantageusement dans un solvant inerte approprié, de préférence le toluène, en présence d'un tamis moléculaire.

17. Procédé suivant la revendication 15, **caractérisé en ce que** le phosphate d'argent de formule Va, Vb ou, respectivement, Vc est remplacé par un phosphate de formule VIa, Vlb ou, respectivement, Vlc : 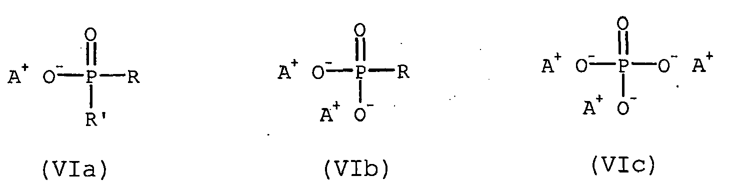 dans lesquelles R et R'. sont définis comme indiqué ci-dessus, et A est H ou R"₄N , R" étant H ou un groupe N-alkyle, cycloalkyle ou aromatique.

## Claims

1. Composition for use as a drug, **characterized in that** it contains, in combination with a physiologically acceptable carrier, an active substance chosen from the group consisting of:
(α) mono(α-D-mannopyranosyl-1) phosphates of formula I: 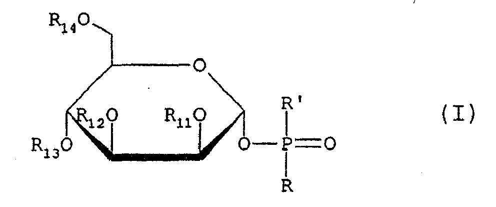 in which
R₁₁, R₁₂, R₁₃ and R₁₄, identical or different, each represent the hydrogen atom or an OH-protective group,
R and R', identical or different, each represent
- a C₆-C₁₀ aryloxy group capable of being substituted by one or more C₁-C₅ alkyl, C₁-C₅ alkoxy, halogen, CF₃ and/or nitro groups.
- an arylalkyleneoxy group, in which the alkylene radical is at C₁-C₅, and the aryl radical, which is at C₆-C₁₀, is capable of being substituted by one or more C₁-C₅ alkyl, C₁-C₅ alkoxy, halogen, CF₃ and/or nitro groups,
- a group with the structure:
-O-CH(CH₃)-O-CO-alkyl, or
-O-CH(CH₃)-O-CO-O-alkyl
in which the alkyl radical is at C₁-C₅,
- an -O-CH₂-CH(OH)-CH₂OH group, in which the OH groups can be protected,
- an OB radical, in which B is an ethylenically unsaturated C₂-C₂₁ aliphatic radical with a linear or branched hydrocarbon chain, or a C₅-C_{2I} cycloaliphatic radical,
- an amino acid group with the structure VIIa: 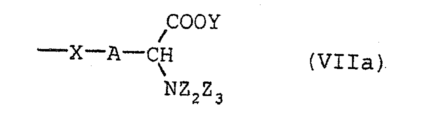 in which
X is -0-, -S- or -NZ₁-,
Y represents H or a C₂-C₅ alkyl group,
A is an alkylene, phenylene or phenylalkylene group (in which each alkylene group is at C₁-C₅),
Z₁ is H, a C₁-C₅ alkyl group or an N-projective group,
Z₂ and Z₃, identical or different, each represent H, a C₁-C₅ alkyl group, or an N-protective group;
- an amino acid group with the structure VIIb: 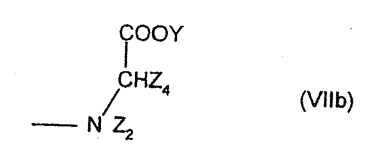 in which
Y represents H or a C₂-C₅ alkyl group,
Z₄ is an alkylene, phenylene or phenylalkylene group (in which each alkylene group is at C₁-C₅),
Z₂ represents H, a C₁-C₅ alkyl group, or an N-protective group;
(β) di(α-D-mannopyranosyl-1) phosphates of formula II: 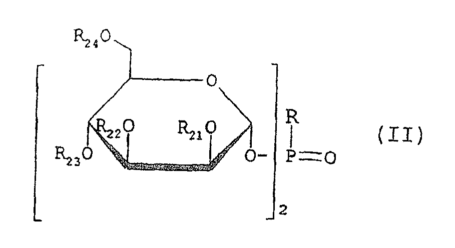 in which
R₂₁, R₂₂, R₂₃, and R₂₄, identical or different, each represent the hydrogen atom or an OH-protective group, and R represents
- an OH group,
- a C₁-C₂₀ alkoxy group,
- a C₆-C₁₀ aryloxy group capable of being substituted by one or more C₁-C₅ alkyl, C₁-C₅ alkoxy, halogen, CF₃ and/or nitro groups,
- an arylalkyleneoxy group, in which the alkylene radical is at C₁-C₅, and the aryl radical, which is at C₆-C₁₀, is capable of being substituted by one or more C₁-C₅ alkyl, C₁-C₅ alkoxy, halogen, CF₃ and/or nitro groups,
- a group with the structure:
-O-CH(Q)-O-CO-alkyl, or
-O-CH(Q)-O-CO-O-alkyl
in which Q is H or CH₃, and the alkyl radical is at C₁-C₅,
- an -O-CH₂-CH(OH)-CH₂OH group, in which the OH groups can be protected,
- an OB radical, in which B is an ethylenically unsaturated C₂-C₂₁ aliphatic radical with a linear or branched hydrocarbon chain, or a C₅-C₂₁ cycloaliphatic radical, or
- an amino acid group with the structure VIIa: 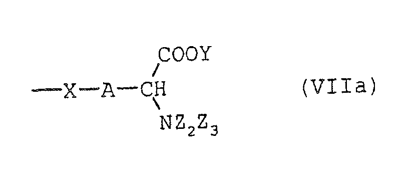 in which
X is -0-, -S- or -NZ₁,
Y represents H or a C₂-C₅ alkyl group,
A is an alkylene, phenylene or phenylalkylene group (in which each alkylene group is at C₁-C₅) , Z₁ is H, a C₁-C₅ alkyl group, or an N-protective group, and
Z₂ and Z₃, identical or different, each represent H, a C₁-C₅ alkyl group, or an N-protective group;
- an amino acid group with the structure VIIb: 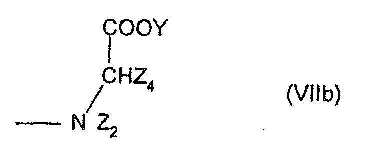 in which
Y represents H or a C₂-C₅ alkyl group,
Z₄ alkylene, phenylene or phenylalkylene group (in which each alkylene group is at C₁-C₅),
Z₂ represents H, a C₁-C₅ alkyl group, or an N-protective group;
(γ) tri(α-D-mannopyranosyl-1) phosphates of formula III: 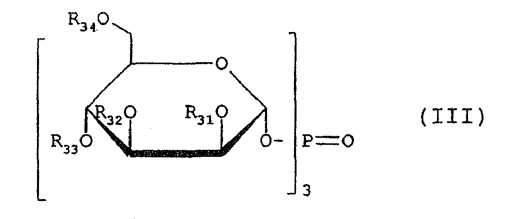 in which R₃₁, R₃₂, R₃₃ and R₃₄, identical or different, each represent a hydrogen atom or an OH-protective group, and
(δ) mixtures thereof.

2. Composition according to claim 1, for use as a drug to treat CDG syndrome type-I.

3. Composition according to claim 2, for use as a drug to treat CDG syndrome type-Ia.

4. Composition according to any one of claims 1 to 3, **characterized in that** said OH-protective group of the hydroxyl groups in positions 2, 3, 4 and 5 of the mannopyranosyl ring is an acyl group.

5. Composition according to any one of claims 1 to 3, **characterized in that**, in formulas I, II and III, the OH-protective group of the OH functions in positions 2, 3, 4 and 6 of the mannosyl ring is a C₂-C₆ aliphatic acyl group.

6. Use of a derivative of (mannosyl-1) phosphate, said use being **characterized in that** a substance involved as an intracellular source of Man-1 P is used, which is chosen from the group consisting of the compounds:
(α) mono(α-D-mannopyranosyl-1) phosphates of formula I,
(β) di(α-D-mannopyranosyl-1) phosphates of formula II,
(γ) tri(α-D-mannopyranosyl-1) phosphates of formula III, and
(δ) mixtures thereof, according to any one of claims 1 to 3, for preparing a drug intended for therapeutic use to treat CDG syndrome type-I, and in particular CDG syndrome type-Ia.

7. Derivative of mannosyl-1 phosphate, for use as a drug, **characterized in that** it is chosen from the group consisting of:
(α) mono(α-D-mannopyranosyl-1) phosphates of formula I: 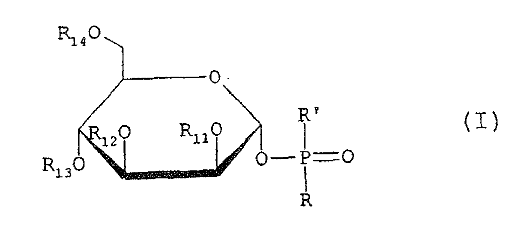 in which
R₁₁, R₁₂, R₁₃ and R₁₄, identical or different, each represent the hydrogen atom or an OH-protective group,
R and R', identical or different, each represent
- a C₆-C₁₀ aryloxy group (in particular phenoxy, 1-naphthyloxy or 2-naphthyloxy) capable of being substituted by one or more C₁-C₅ alkyl, C₁-C₅ alkoxy, halogen, CF₃ and/or nitro groups.
- an arylalkyleneoxy group (in particular OCH₂CH₂C₆H₅, OCH₂C₆H₅, 1-naphthylmethyloxy or 2-naphthylmethyloxy), in which the alkylene radical is at C₁-C₅, and the aryl radical, which is at C₆-C₁₀, is capable of being substituted by one or more C₁-C₅ alkyl, C₁-C₅ alkoxy, halogen, CF₃ and/or nitro groups,
- a group with the structure:
-O-CH(CH₃)-O-CO-alkyl, or
-O-CH(CH₃)-O-CO-O-alkyl
in which the alkyl radical is at C₁-C₅,
- an -O-CH₂-CH(OH)-CH₂OH group, in which the OH groups can be protected,
- an OB radical, in which B is an ethylenically unsaturated C₂-C₂₁ aliphatic radical with a linear or branched hydrocarbon chain, or a C₅-C_{2I} cycloaliphatic radical,
- an amino acid group with the structure VIIa: 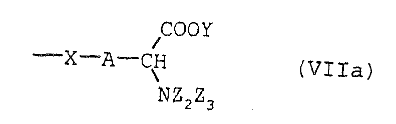 in which
X is -O-, -S- or -NZ,-,
Y represents H or a C₂-C₅ alkyl group,
A is an alkylene, phenylene or phenylalkylene group (in which each alkylene group is at C₁-C₅),
Z₁ is H, a C₁-C₅ alkyl group or an N-protective group,
Z₂ and 2₃, identical or different, each represent H, a C₁-C₅ alkyl group, or an N-protective group;
- an amino acid group with the structure VIIb: 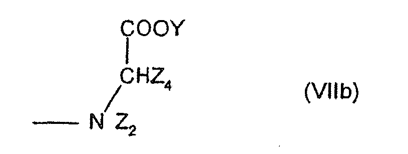 in which
Y represents H or a C₂-C₅ alkyl group,
Z₄ is an alkylene, phenylene or phenylalkylene group (in which each alkylene group is at C₁-C₅) ,
Z₂ represents H, a C₁-C₅ alkyl group, or an N-protective group;
(β) di(α-D-mannopyranosyl-1) phosphates of formula II: 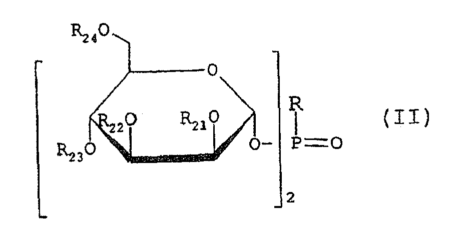 in which
R₂₁, R₂₂, R₂₃, and R₂₄, identical or different, each represent the hydrogen atom or an OH-protective group, and R represents
- an OH group,
- a C₁-C₂₀ alkoxy group (preferably C₁-C₅),
- a C₆-C₁₀ aryloxy group capable of being substituted by one or more C₁-C₅ alkyl, C₁-C₅ alkoxy, halogen, CF₃ and/or nitro groups,
- an arylalkyleneoxy group (in particular benzyloxy, phenylethyloxy, 1-naphthylmethyloxy or 2-naphthylmethyloxy, in which the alkylene radical is at C1-C5, and the aryl radical, which is at C₆-C₁₀, is capable of being substituted by one or more C₁-C₅ alkyl, C₁-C₅ alkoxy, halogen, CF₃ and/or nitro groups,
- a group with the structure:
-O-CH(Q)-O-CO-alkyl, or
-O-CH(Q)-0-CO-O-alkyl
in which Q is H. or CH₃, and the alkyl radical is at C₁-C₅,
- an -O-CH₂-CH(OH)-CH₂OH group, in which the OH groups can be protected,
- an OB radical, in which B is an ethylenically unsaturated C₂-C₂₁ aliphatic radical with a linear or branched hydrocarbon chain, or a C₅-C₂₁ cycloaliphatic radical, or
- an amino acid group with the structure VII: 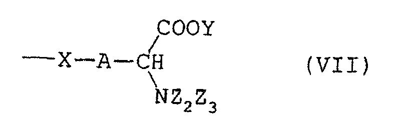 in which
X is -O-, -S- or -NZ₁,
Y represents H or a C₂-C₅ alkyl group,
A is an alkylene, phenylene or phenylalkylene group (in which each alkylene group is at C₁-C₅),
Z₁ is H, a C₁-C₅ alkyl group, or an N-protective group, and
Z₂ and Z₃, identical or different, each represent H, a C₁-C₅ alkyl group, or an N-protective group;
- an amino acid group with the structure VIIb: 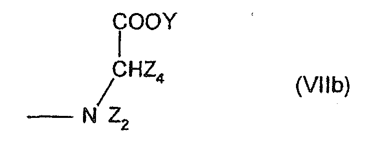 in which
Y represents H or a C₂-C₅ alkyl group,
Z₄ alkylene, phenylene or phenylalkylene group (in which each alkylene group is at C₁-C₅),
Z₂ represents H, a C₁-C₅ alkyl group, or an N-protective group;
(γ) tri(α-D-mannopyranosyl-1) phosphates of formula III': 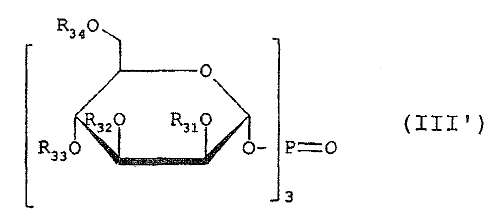 in which R₃₁, R₃₂, R₃₃ and R₃₄, identical or different, each represent a hydrogen atom or an OH-protective group, and
(δ) mixtures thereof.

8. Derivative of (α-D-mannosyl-1) phosphate according to claim 7, for use as a drug to treat CDG syndrome type-I.

9. Derivative of (α-D-mannosyl-1) phosphate according to claim 8, for use as a drug to treat CDG syndrome type-Ia.

10. Derivative of (α-D-mannosyl-1) phosphate according to any one of claims 7 to 9, **characterized in that**, in formulas I and II, the OH-protective group of the OH functions in positions 2, 3, 4 and 6 of the mannosyl ring is a C₂-C₆ aliphatic acyl group, and **in that**, in formula III', the OH-protective group of the OH functions in positions 2, 3, 4 and 6 of the mannosyl ring is a C₃-C₆ aliphatic acyl group.

11. Derivative of (α-D-mannosyl-1) phosphate according to any one of claims 7 to 9, **characterized in that** the R (or R') group is an OB group in which B is an ethylenically unsaturated C₂-C₂₁ aliphatic radical capable of comprising one or more C=C double bonds, with a linear or branched hydrocarbon chain, or a C₅-C₂₁ cycloaliphatic radical, in which OB is in particular an -O-CH₂-CH=C(CH₃)₂, O-(CH₂)₂-CH=C(CH₃)₂ or a terpeneoxy group.

12. Derivative of (α-D-mannosyl-1) phosphate according to any one of claims 7 to 9 or 11, **characterized in that** the R (or R') group is an OB group = terpeneoxy, in which the terpene portion is cyclic or acyclic, and OB is in particular a farnesyloxy or geranyloxy group.

13. Derivative of (α-D-mannosyl-1) phosphate according to any one of claims 7 to 9, **characterized in that** the R (or R') group is a group of structure VII obtained from an amino acid comprising an amine or lateral hydroxyl function.

14. Derivative (α-D-mannosyl-1) phosphate according to any one of claims 7 to 9, **characterized in that** the R (or R') group represents, in formula I or II:
(α) a phenoxy or 1-naphthyloxy group,
(β) a benzyloxy or 1-naphthylmethoxy group,
(γ) an -O-CH (Q)-O-CO-O-(C₁-C₅) alkyl, in which Q is H or CH₃,
(δ) an -O-CH₂-CH(OH)-CH₂OH group, in which the OH groups can be protected,
(ε) a εLys, pTyr, βSer or βThr group, of which the structures (in which the NH₂ and COOH groups can be protected) are as follows:
εLys: -NH-(CH₂)₄-CH (NH₂) COOH
pTyr: -(P-O)-C₆H₄-CH₂-CH(NH₂)COOH
βSer: -O-CH₂-CH(NH₂)COOH
βThr : -O-CH(CH₃)-CH(NH₂) COOH, or
(ζ) a group
-NH-(CH₂)₃-CH (NH₂) COOH or
-NH-(CH₂)₂-CH (NH₂) COOH,
in which the NH₂ and COOH functions can be protected.
R can also represent, in formula II:
(η) a -O-CH(Q)-O-CO-(C₁-C₅) alkyl group, in which Q is H or CH₃.

15. Process for preparing a compound of formula I, II or III' according to any one of claims 7 to 9, said process being **characterized in that** it includes:
(a) the reaction of a 1-bromo-mannopyranose of formula (Iva): 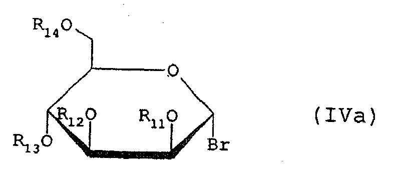 in which R₁₁, R₁₂, R₁₃ and R₁₄ are defines as indicated above, with a mono-silver phosphate of formula (Va): 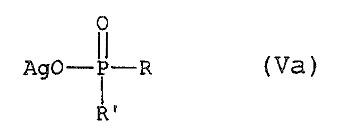 in which R and R' are defined as indicated above,
in order to obtain a mono(α-D-mannopyranosyl-1) phosphate compound of formula I;
(b) the reaction of a 1-bromo-mannopyranose of formula (IVb): 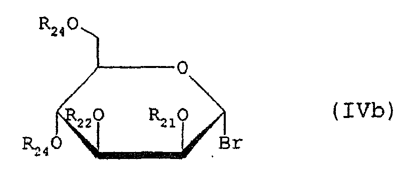 in which R₂₁, R₂₂, R₂₃ and R₂₄ are defines as indicated above, with a di-silver phosphate of formula (Vb): 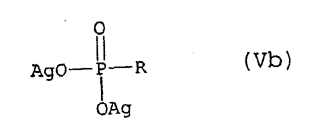 in which R is defined as indicated above,
in order to obtain a di(α-D-mannopyranosyl-1) phosphate compound of formula II; or
(c) the reaction of a 1-bromo-mannopyranose of formula (IVc): 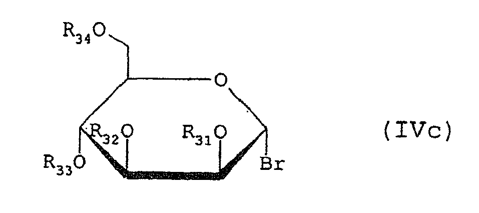 in which R₃₁, R₃₂, R₃₃ and R₃₄, identical or different, each represent an OH-protective group that is a C₃-C₆ acyl group, with a tri-silver phosphate of formula (Vc): 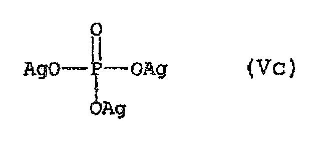 in order to obtain a tri(α-D-mannopyranosyl-1) phosphate compound of formula III'.

16. Process according to claim 15, **characterized in that** the reaction of Iva with Va, the reaction of IVb with Vb or the reaction of IVc with Vc is implemented at a temperature of 15 to 40°C, preferably at room temperature (15-25°C), advantageously in a suitable inert solvent, preferably toluene, in the presence of a molecular sieve.

17. Process according to claim 15, **characterized in that** the silver phosphate of formula Va, Vb or Vc, respectively, is replaced by a phosphate of formula Via, VIb or VIc, respectively: 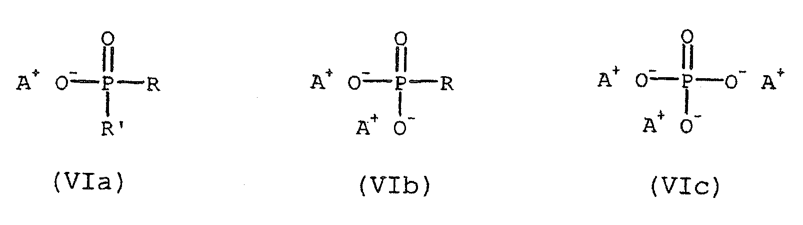 in which R and R' are defined as indicated above, and A is H or R"₄N, R" being H or an N-alkyl, cycloalkyl or aromatic group.

## Patentansprüche

1. Zusammensetzung zur Verwendung als Medikament, **dadurch gekennzeichnet, dass** sie in Kombination mit einem physiologisch verträglichen Exzipienten enthält, einen Wirkstoff, ausgewählt aus der Gruppe, bestehend aus:
(α) den Mono-(α-D-mannopyranosyl-1)-phosphaten der Formel I: 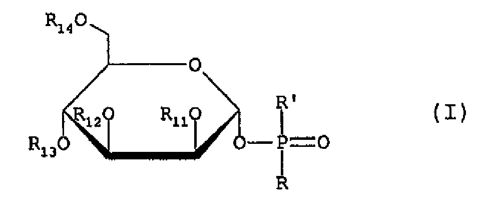 worin
R₁₁, R₁₂, R₁₃ und R₁₄, die gleich oder verschieden sind, jeweils das Wasserstoff-Atom oder eine OH-Schutz-Gruppe wiedergeben,
R und R', die gleich oder verschieden sind, jeweils wiedergeben
- eine C₆-C₁₀-Aryloxy--Gruppe, die mit einer oder mehreren C₁-C₅-Alkyl-, C₁-C₅-Alkoxy-, Halogen-, CF₃- und/oder Nitro-Gruppen substituiert sein kann,
- eine Aryl-alkylenoxy-Gruppe, wobei der Akylen-Rest, der C₁-C₅ ist, und der Aryl-Rest, der C₆-C₁₀ ist, mit einer oder mehreren C₁-C₅-Alkyl-, C₁-C₅-Alkoxy-, Halogen-, CF₃- und/oder Nitro-Gruppen substituiert sein können,
- eine Gruppe der Struktur:
-O-CH (CH₃) -O-CO-Alkyl, oder
-O-CH (CH₃) -O-CO-O-Alkyl
wobei der Alkyl-Rest C₁-C₅ ist,
- eine Gruppe -O-CH₂-CH(OH)-CH₂OH, worin die OH-Gruppen geschützt sein können,
- einen Rest OB, worin B einen ethylenisch ungesättigten aliphatischen C₂-C₂₁-Rest, der in der Kohlenwasserstoff-Kette linear oder verzweigt ist, oder einen cyclo-aliphatischen C₅-C₂₁-Rest darstellt,
- eine Aminosäure-Gruppe der Struktur VIIa: 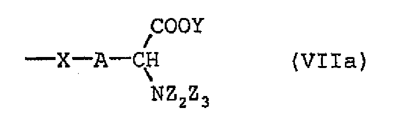 worin
X -O-, -S- oder -NZ₁- darstellt,
Y H oder eine C₂-C₅-Alkyl-Gruppe wiedergibt,
A eine Akylen-, Phenylen-- oder Phenylalkylen-Gruppe darstellt, (wobei jede Alkylen-Gruppe C₁-C₅ ist),
Z₁ H, eine C₁-C₅-Alkyl-Gruppe oder eine N-Schutz-Gruppe darstellt,
Z₂ und Z₃, die gleich oder verschieden sind, jeweils H, eine C₁-C₅-Alkyl-Gruppe oder eine N-Schutz-Gruppe wiedergeben;
- eine Aminosäure-Gruppe der Struktur VIIb: 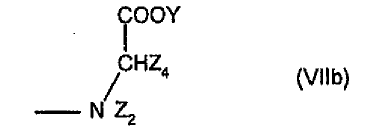 worin
Y H oder eine C₂-C₅-Alkyl-Gruppe wiedergibt,
Z₄ eine Alkylen-, Phenylen- oder Phenylalkylen-Gruppe darstellt, (wobei jede Alkylen-Gruppe C₁-C₅ ist),
Z₂ H, eine C₁-C₅-Alkyl-Gruppe oder eine N-Schutz-Gruppe wiedergibt;
(β) den Di-(α-D-mannopyranosyl-1)-phosphaten der Formel II: 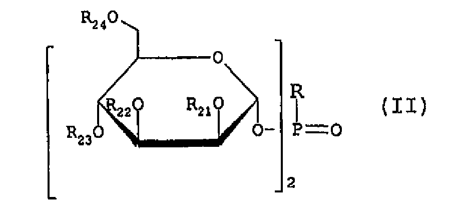 worin
R₂₁, R₂₂, R₂₃ und R₂₄, die gleich oder verschieden sind, jeweils das Wasserstoff-Atom oder eine OHSchutz-Gruppe wiedergeben, und
R wiedergibt
- eine OH-Gruppe,
- eine C₁-C₂₀-Alkoxy-Gruppe,
- eine C₆-C₁₀-Aryloxy-Gruppe, die mit einer oder mehreren C₁-C₅-Alkyl-, C₁-C₅-Alkoxy-, Halogen-, CF₃- und/oder Nitro-Gruppen substituiert sein kann,
- eine Aryl-alkylenoxy-Gruppe, wobei der Alkylen-Rest, der C₁-C₅ ist, und der Aryl-Rest, der C₆-C₁₀ ist, mit einer oder mehreren C₁-C₅-Alkyl-, C₁-C₅-Alkoxy-, Halogen-, CF₃- und/oder Nitro-Gruppen substituiert sein können,
- eine Gruppe der Struktur:
-O-CH (Q)-O-CO-Alkyl, oder
-O-CH (Q)-O-CO-O-Alkyl,
wobei Q H oder CH₃ darstellt und der Alkyl-Rest C₁-C₅ ist,
- eine Gruppe -O-CH₂-CH(OH)-CH₂OH, worin die OH-Gruppen geschützt sein können,
- einen Rest OB, worin B einen ethylenisch ungesättigten aliphatischen C₂-C₂,-Rest, der in der Kohlenwasserstoff-Kette linear oder verzweigt ist, oder einen cyclo-aliphatischen C₅-C₂₁-Rest darstellt, oder
- eine Aminosäure-Gruppe der Struktur VIIa: 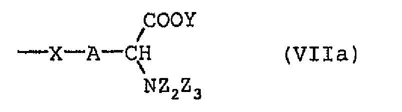 worin
X -O-, -S- oder -NZ₁- darstellt,
Y H oder eine C₂-C₅-Alkyl-Gruppe wiedergibt,
A eine Alkylen-, Phenylen- oder Phenylalkylen-Gruppe darstellt, (wobei jede Alkylen-Gruppe C₁-C₅ ist),
Z₁ H, eine C₁-C₅-Alkyl-Gruppe oder eine N-Schutz-Gruppe darstellt, und
Z₂ und Z₃, die gleich oder verschieden sind, jeweils H, eine C₁-C₅-Alkyl-Gruppe oder eine N-Schutz-Gruppe wiedergeben;
- eine Aminosäure-Gruppe der Struktur VIIb: 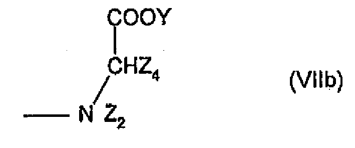 worin
Y H oder eine C₂-C₅-Alkyl-Gruppe wiedergibt,
Z₄ eine Alkylen-, Phenylen- oder Phenyl-alkylen-Gruppe darstellt, (wobei jede Alkylen-Gruppe C₁-C₅ ist),
Z₂ H, eine C₁-C₅-Alkyl-Gruppe oder eine N-Schutz-Gruppe wiedergibt;
(γ) den Tri-(α-D-mannopyranosyl-1)-phosphaten der Formel III: 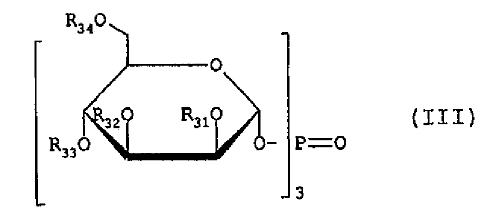 worin
R₃₁, R₃₂, R₃₃ und R₃₄, die gleich oder verschieden sind, jeweils ein Wasserstoff-Atom oder eine OH-Schutz-Gruppe wiedergeben, und
(δ) Gemischen davon.

2. Zusammensetzung nach Anspruch 1, zur Verwendung als Medikament gegen das CDG-I-Syndrom.

3. Zusammensetzung nach Anspruch 2 zur Verwendung als Medikament gegen das CDG-Ia-Syndrom.

4. Zusammensetzung nach einem der Ansprüche 1 bis 3, **dadurch gekennzeichnet, dass** die OH-Schutz-Gruppe der HydroxylGruppen in den Positionen 2, 3, 4 und 5 des Mannopyranosyl-Rings eine Acyl-Gruppe darstellt.

5. Zusammensetzung nach einem der Ansprüche 1 bis 3, **dadurch gekennzeichnet, dass** in den Formeln I, II und III die OH-Schutz-Gruppe für die OH-Funktionen an den Positionen 2, 3, 4 und 6 des Mannosyl--Rings eine aliphatische C₂-C₆-Acyl-Gruppe darstellt.

6. Verwendung eines (Mannosyl-1)-phosphat-Derivats, wobei die Verwendung **dadurch gekennzeichnet ist, dass** ein Stoff verwendet wird, der als eine intrazelluläre Quelle von Man-1 P wirkt, welcher ausgewählt ist aus der Gruppe, bestehend aus den Verbindungen
(α) Mono-(α-D-mannopyranosyl-1)-phosphate der Formel I,
(β) Di-(α-D-mannopyranosyl-1)-phosphate der Formel II,
(γ) Tri-(α-D-mannopyranosyl-1)-phosphate der Formel III, und
(δ) Gemische davon,
nach einem der Ansprüche 1 bis 3,
zur Herstellung eines Medikaments, das zur therapeutischen Verwendung gegen das CDG-I Syndrom, und insbesondere gegen das CDG-Ia Syndrom, vorgesehen ist.

7. Mannosyl-1-phosphat-Derivat zur Verwendung als Medikament, **dadurch gekennzeichnet, dass** es ausgewählt ist aus der Gruppe, bestehend aus:
(α) den Mono-(α-D-mannopyranosyl-1)-phosphaten der Formel I: 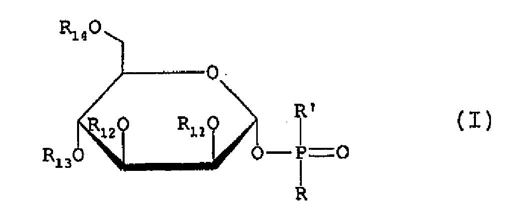 worin
R₁₁, R₁₂, R₁₃ und R₁₄, die gleich oder verschieden sind, jeweils das Wasserstoff-Atom oder eine OH-Schutz-Gruppe wiedergeben,
R und R', die gleich oder verschieden sind, jeweils wiedergeben
- eine C₆-C₁₀-Aryloxy-Gruppe (insbesondere Phenoxy, 1-Naphthyloxy oder 2-Naphthyloxy), die mit einer oder mehreren C₁-C₅-Alkyl-, C₁-C₅-Alkoxy-, Halogen-, CF₃- und/oder Nitro-Gruppen substituiert sein kann,
- eine Aryl-alkylenoxy-Gruppe (insbesondere OCH₂CH₂C₆H₅, OCH₂C₆H₅, 1-Naphthyl-methyloxy oder 2-Naphthyl-methyloxy) , wobei der Alkylen-Rest, der C₁-C₅ ist, und der Aryl-Rest, der C₆-C₁₀ ist, mit einer oder mehreren C₁-C₅-Alkyl-, C₁-C₅-Alkoxy-, Halegen-, CF₃- und/oder Nitro-Gruppen substituiert sein können,
- eine Gruppe der Struktur:
-O-CH (CH₃) -O-CO-Alkyl, oder
-O-CH (CH₃) -O-CO-O-Alkyl,
wobei der Alkyl-Rest C₁-C₅ ist,
- eine Gruppe -O-CH₂-CH(OH)-CH₂OH, worin die OH-Gruppen geschützt sein können,
- einen Rest OB, worin B einen ethylenisch ungesättigten aliphatischen C₂-C₂,-Rest, der in der Kohlenwasserstoff-Kette linear oder verzweigt ist, oder einen cyclo-aliphatischen C₅-C₂₁-Rest darstellt, oder
- eine Aminosäure-Gruppe der Struktur VIIa: 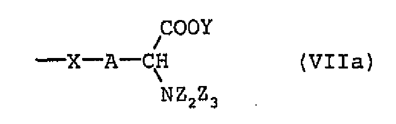 worin
X -O-, -S- oder -NZ₁- darstellt,
Y H oder eine C₂-C₅-Alkyl-Gruppe wiedergibt,
A eine Alkylen-, Phenylen- oder Phenylalkylen-Gruppe darstellt, (wobei jede Alkylen-Gruppe C₁-C₅ ist),
Z₁ H, eine C₁-C₅-Alkyl-Gruppe oder eine N-Schutz-Gruppe darstellt, und
Z₂ und Z₃, die gleich oder verschieden sind, jeweils H, eine C₁-C₅-Alkyl-Gruppe oder eine N-Schutz-Gruppe wiedergeben;
- eine Aminosäure-Gruppe der Struktur VIIb: 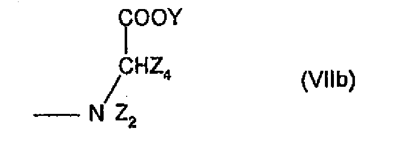 worin
Y H oder eine C₂-C₅-Alkyl-Gruppe wiedergibt,
Z₄ eine Alkylen-, Phenylen- oder Phenylalkylen-Gruppe darstellt, (wobei jede Alkylen-Gruppe C₁-C₅ ist),
Z₂ H, eine C₁-C₅-Alkyl-Gruppe oder eine N-Schutz-Gruppe wiedergibt;
(β) den Di-(α-D-mannopyranosyl-1)-phosphaten der Formel II: 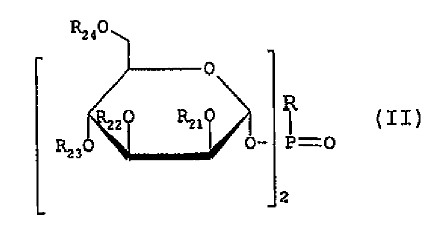 worin
R₂₁, R₂₂, R₂₃ und R₂₄, die gleich oder verschieden sind, jeweils das Wasserstoff-Atom oder eine OH-Schutz-Gruppe wiedergeben, und
R wiedergibt
- eine OH-Gruppe,
- eine C₁-C₂₀-Alkoxy-Gruppe (vorzugsweise C₁-C₅) ,
- eine C₆-C₁₀-Aryloxy-Gruppe, die mit einer oder mehreren C₁-C₅-Alkyl-, C₁-C₅-Alkoxy-, Halogen-, CF₃- und/oder Nitro-Gruppen substituiert sein kann,
- eine Aryl-alkylenoxy-Gruppe (insbesondere Benzyloxy, Phenyl-ethyl-oxy, 1-Naphthyl-methyl-oxy oder 2-Naphthyl-methyl-oxy), wobei der Akylen-Rest, der C₁-C₅ ist, und der Aryl-Rest, der C₆-C₁₀ ist, mit einer oder mehreren C₁-C₅-Alkyl-, C₁-C₅-Alkoxy-, Halogen-, CF₃- und/oder Nitro-Gruppen substituiert sein können,
- eine Gruppe der Struktur:
-O-CH (Q) -O-CO-Alkyl, oder
-O-CH(Q)-O-CO-O-Alkyl,
wobei Q H oder CH₃ darstellt und der Alkyl-Rest C₁-C₅ ist,
- eine Gruppe -O-CH₂-CH(OH)-CH₂OH, worin die OH-Gruppen geschützt sein können,
- einen Rest OB, worin B einen ethylenisch ungesättigten aliphatischen C₂-C₂₁-Rest, der in der Kohlenwasserstoff-Kette linear oder verzweigt ist, oder einen cyclo-aliphatischen C₅-C₂₁-Rest darstellt, oder
- eine Aminosäure-Gruppe der Struktur VII: 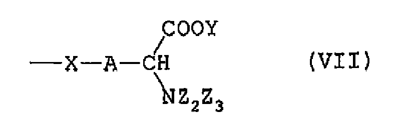 worin
X -O-, -S- oder -NZ₁- darstellt,
Y H oder eine C₂-C₅-Alkyl-Gruppe wiedergibt,
A eine Alkylen-, Phenylen- oder Phenylalkylen-Gruppe darstellt, (wobei jede Alkylen-Gruppe C₁-C₅ ist),
Z₁ H, eine C₁-C₅-Alkyl-Gruppe oder eine N-Schutz-Gruppe darstellt, und
Z₂ und Z₃, die gleich oder verschieden sind, jeweils H, eine C₁-C₅-Alkyl-Gruppe oder eine N-Schutz-Gruppe wiedergeben;
- eine Aminosäure-Gruppe der Struktur VIIb: 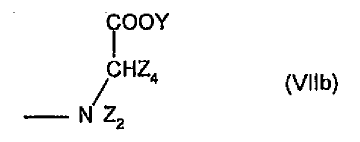 worin
Y H oder eine C₂-C₅-Alkyl-Gruppe wiedergibt,
Z₄ eine Alkylen-, Phenylen- oder Phenylalkylen-Gruppe darstellt, (wobei jede Alkylen-Gruppe C₁-C₅ ist),
Z₂ H, eine C₁-C₅-Alkyl-Gruppe oder eine N-Schutz-Gruppe wiedergibt;
(γ) den Tri-(α-D-mannopyranosyl-1)-phosphaten der Formel III': 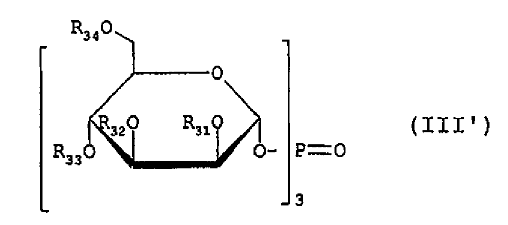 worin
R₃₁, R₃₂, R₃₃ und R₃₄, die gleich oder verschieden sind, jeweils eine OH-Schutz-Gruppe mit mindestens drei Kohlenstoff-Atomen wiedergeben, und
(δ) Gemischen davon.

8. (α-D-Mannosyl-1)-phosphat-Derivat nach Anspruch 7, zur Verwendung als Medikament gegen das CDG-I-Syndrom.

9. (α-D-Mannasyl-1)-phosphat-Derivat nach Anspruch 8, zur Verwendung als Medikament gegen das CDG-Ia-Syndrom.

10. (α-D-Mannosyl-1)-phosphat-Derivat nach einem der Ansprüche 7 bis 9, **dadurch gekennzeichnet, dass** in den Formeln I und II, die OH-Schutz-Gruppe für die OH-Funktionen an den Positionen 2, 3, 4 und 6 des Mannosyl-Rings eine aliphatische C₂-C₆-Acyl-Gruppe darstellt, und dass in der Formel III' die OH-Schutz-Gruppe für die OH-Funktionen an den Positionen 2, 3, 4 und 6 des Mannosyl-Rings eine aliphatische C₃-C₆-Acyl-Gruppe darstellt.

11. (α-D-Mannosyl-1)-phosphat-Derivat nach einem der Ansprüche 7 bis 9, **dadurch gekennzeichnet, dass** die Gruppe R (oder R') eine Gruppe OB darstellt, worin B einen ethylenisch ungesättigten, aliphatischen, in der KohlenwasserstoffKette linearen oder verzweigten C₂-C₂₁-Rest, der eine oder mehrere Doppelbindungen C=C enthalten kann, oder einen cyclo-aliphatischen C₅-C₂₁-Rest darstellt, wobei OB insbesondere eine Gruppe -O-CH₂-CH=C(CH₃)₂, O-(CH₂)₂-CH=C(CH₃)₂ oder eine Terpenoxy-Gruppe darstellt.

12. (α-D-Mannosyl-1)-phosphat-Derivat nach einem der Ansprüche 7 bis 9 oder 11, **dadurch gekennzeichnet, dass** die Gruppe R (oder R') eine Gruppe OB = Terpenoxy darstellt, worin der Terpen-Teil cyclisch oder acyclisch ist, wobei OB insbesondere eine Farnesyloxy- oder Geranyloxy-Gruppe darstellt.

13. (α-D-Mannosyl-1)-phosphat-Derivat nach einem der Ansprüche 7 bis 9, **dadurch gekennzeichnet, dass** die Gruppe R (oder R') eine Gruppe der Struktur VII darstellt, die von einer Aminosäure stammt, die eine seitliche Amin- oder Hydroxyl-Funktion enthält.

14. (α-D-Mannosyl-1)-phosphat-Derivat nach einem der Ansprüche 7 bis 9, **dadurch gekennzeichnet, dass** die Gruppe R (oder R') in der Formel I oder II wiedergibt:
(α) eine Phenoxy oder 1-Naphthyloxy-Gruppe,
(β) eine Benzyloxy-- oder 1-Naphthylmethoxy-Gruppe,
(γ) eine Gruppe -O-CH(Q)-O-CO-O-(C1-C5)-Alkyl, worin Q H oder CH₃ darstellt,
(δ) eine Gruppe -O-CH₂-CH(OH)-CH₂OH, worin die OH-Gruppen geschützt sein können,
(ε) eine Gruppe εLys, pTyr; βSer oder βThr, deren Strukturen (wobei die Gruppen NH₂ und COOH geschützt sein können) die nachstehenden sind:
εLyS : -NH-(CH₂)₄-CH(NH₂)COOH,
pTyr: -(p-O)-C₆H₄-CH₂-CH (NH₂) COOH,
βSer : -O-CH₂-CH(NH₂)COOH, und
βThr : -O-CH(CH₃)-CH(NH₂) COOH, oder
(ζ) eine Gruppe
-NH- (CH₂) ₃-CH (NH₂) COOH oder
-NH-(CH₂)₂-CH (NH₂)COOH,
wobei die Funktionen NH₂ und COOH geschützt sein können,
wobei R in der Formel II auch wiedergeben kann:
(η) eine Gruppe -O-CH(Q)-O-CO-(C₁-C₅)-Alkyl, worin Q H oder CH₃ bedeutet.

15. Verfahren zur Herstellung einer Verbindung der Formel I, II oder III' nach einem der Ansprüche 7 bis 9, wobei das Verfahren **dadurch gekennzeichnet ist, dass** es umfasst
(a) die Reaktion von einer 1-Brom-mannopyranose der Formel (IVa) : 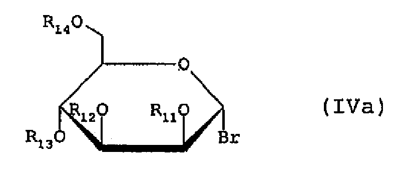 worin R₁₁, R₁₂, R₁₃ und R₁₄ wie vorstehend ausgewiesen definiert sind, mit einem Mono-silber-phosphat der Formel (Va): 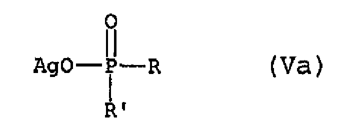 worin R und R' wie vorstehend ausgewiesen definiert sind,
um eine Mono-(α-D-mannopyranosyl-1)-phosphat-Verbindung der Formel I zu erhalten;
(b) die Reaktion von einer 1-Brom-mannopyranose der Formel (IVb): 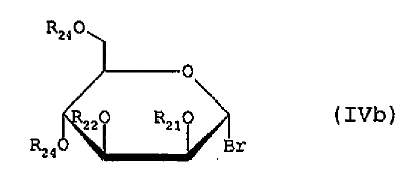 worin R₂₁, R₂₂, R₂₃ und R₂₄ wie vorstehend ausgewiesen definiert sind,
mit einem Di-silber-phosphat der Formel (Vb) : 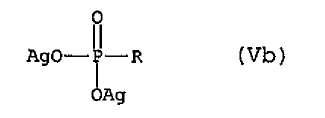 worin R wie vorstehend ausgewiesen definiert ist,
um eine Di-(α-D-mannopyranosyl-1)-phosphat-Verbindung der Formel 11 zu erhalten; oder
(c) die Reaktion von einer 1-Brom-mannopyranose der Formel (IVc): 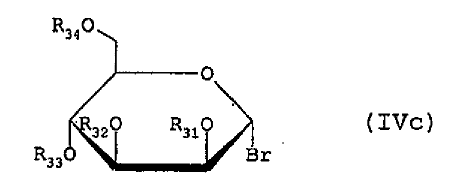 worin R₃₁, R₃₂, R₃₃ und R₃₄, die gleich oder verschieden sind, jeweils eine OH-Schutz-Gruppe wiedergeben, die eine C₃-C₆-Acyl-Gruppe darstellt,
mit einem Tri-silber-phosphat der Formel (Vc) : 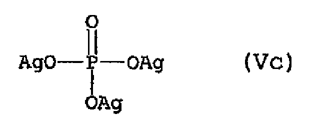
um eine Tri-(α-D-mannopyranosyl-1)-phosphat-Verbindung der Formel III' zu erhalten.

16. Verfahren nach Anspruch 15, **dadurch gekennzeichnet, dass** die Reaktion von IVa mit Va, die Reaktion von IVb mit Vb oder die Reaktion von IVc mit Vc bei einer Temperatur von 15 bis 40°C, vorzugsweise bei Umgebungstemperatur (15-25°C), in vorteilhafter Weise in einem geeigneten inerten Lösungsmittel, vorzugsweise Toluol, in Gegenwart von einem Molekularsieb, ausgeführt wird.

17. Verfahren nach Anspruch 15, **dadurch gekennzeichnet, dass** das Silber-phosphat der Formel Va, Vb bzw. Vc durch ein Phosphat der Formel VIa, VIb bzw. VIc ersetzt wird: 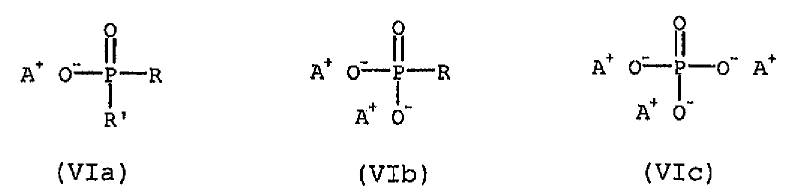 worin R und R' wie vorstehend ausgewiesen definiert sind, und A H oder R" ₄N darstellt, R" H oder eine N-Alkyl-, Cycloalkyl- oder aromatische Gruppe darstellt.
